# EUROPÄISCHE PATENTSCHRIFT

(11) **EP 1 242 129 B1**
(45) Veröffentlichungstag und Bekanntmachung des Hinweises auf die Patenterteilung: **21.01.2004**
(21) Anmeldenummer: 00942031.6
(22) Anmeldetag: 08.06.2000
(51) Int. Cl.: A61L 27/26, A61L 27/40, A61F 2/08, A61F 2/30, A61L 27/38, C12N 5/10, C12N 15/88

(54) **BIOLOGISCHES GELENKKONSTRUKT**
BIOLOGICAL JOINT CONSTRUCT
PRODUIT DE SYNTHESE BIOLOGIQUE POUR ARTICULATION

(30) Priorität: 08.06.1999 DE 19926083
(43) Veröffentlichungstag der Anmeldung: 25.09.2002
(73) Patentinhaber: Universitätsklinikum Freiburg, 79106 Freiburg (DE)
(72) Erfinder: SCHAEFER, Dirk, Johannes, D-79102 Freiburg (DE); KLEMT, Christof, D-79379 Mullheim (DE); STARK, Gerhard, Björn, D-79874 Breitnau (DE); FRIEDL, Hans-Peter, D-79224 Umkirch (DE)
(74) Vertreter: Keller, Günter, Dr.
(86) Internationale Anmeldenummer: PCT/EP2000/005313
(87) Internationale Veröffentlichungsnummer: WO 2000/074741

(56) Entgegenhaltungen:
- EP-A- 0 339 607
- EP-A- 0 657 178
- WO-A-95/22611
- WO-A-96/03160
- WO-A-96/39196
- WO-A-99/21497
- WO-A-99/25396
- WO-A-99/60951
- DE-A- 3 410 631
- DE-A- 3 810 803
- US-A- 5 374 550
- BEGLEY C T ET AL: "Comparative study of the osteoinductive properties of bioceramic, coral and processed bone graft substitutes" BIOMATERIALS,GB,ELSEVIER SCIENCE PUBLISHERS BV., BARKING, Bd. 16, Nr. 15, 1. Oktober 1995 (1995-10-01), Seiten 1181-1185, XP004032874 ISSN: 0142-9612
- GAZIT D. ET AL.: "Engineered pluripotent mesenchymal cells integrate and differentiate in regenerating bone: a novel cell-mediated gene therapy." THE JOURNAL OF GENE MEDICINE, Bd. 1, 25. März 1999 (1999-03-25), Seiten 121-133, XP001010376
- GAO X ET AL: "CATIONIC LIPOSOME-MEDIATED GENE TRANSFER" GENE THERAPY,MACMILLAN PRESS LTD., BASINGSTOKE,GB, Bd. 2, Nr. 10, 1. Dezember 1995 (1995-12-01), Seiten 710-722, XP000749400 ISSN: 0969-7128

## Beschreibung

Die vorliegende Erfindung betrifft ein biologisches Gelenkkonstrukt und Verfahren zu seiner Herstellung sowie Knochengewebe, das transfizierte Zellen enthält und Verfahren zu dessen Herstellung.

Gelenkdefekte können bislang chirurgisch nur durch Gelenkresektion, Gelenkversteifung, alloplastischen Ersatz mit Kunststoff- oder Metallimplantaten bzw. Materialkombinationen daraus, oder durch biologische Implantate behandelt werden. Alloplastische Implantate werden jedoch nicht integriert und lockern sich unter Belastung. Als biologisches Implantat können nur Fremdgewebe, beispielsweise Kniegelenke als sogenannte Allotransplantate verwendet werden. Fremdgewebeverpflanzungen erfordern eine lebenslange Immunsupression mit Gefahren der Weichteiltumorentstehung. Ein autogener biologischer Ersatz kann bisher nur teilweise durch Einzelkomponenten erfolgen. Neben den bekannten chirurgischen Maßnahmen beinhalten wissenschaftliche Ansätze gegenwärtig die Rekonstruktion kleiner Knorpeldefekte durch eine Knorpelzelltransplantation mit einem Knochenhautlappen (Brittberg-Methode). Es gab verschiedene Ansätze, Einzelkomponenten zu rekonstruieren:

Das US-Patent 5,053,050 beschreibt Zusammensetzungen zur Reparatur von Knorpel oder Knochen, wobei Knorpel- oder Knochenzellen in eine biologische, resorbierbare Trägersubstanz eingebracht werden, welche Serum, Fibrinogen und Thrombin enthält. Die US-Anmeldung 5,041,138 beschreibt ein Verfahren zur Herstellung einer Knorpelstruktur durch Besiedelung einer Trägersubstanz mit Knorpelzellen. In ähnlicher Weise betrifft die US-Anmeldung 5,786,217 ein Verfahren zur Herstellung eines vielzelligen Knorpelkonstrukts, worin Knorpelvorläuferzellen auf ein Trägermaterial aufgebracht werden und durch weiteres Kultivieren differenzieren und Knorpelsubstanz bilden. Das US-Patent 5,736,372 beschreibt eine polymere Trägersubstanz, die Chondrozyten enthält und geeignet ist, um in vivo Knorpel strukturen zu bilden. WO 98/42389 offenbart ein biologisches Material zur Behandlung von Knochen- oder Knorpeldefekten umfassend Periost und knochen- oder knorpelbildende Zellen. WO 97/46665 offenbart ein Implantat umfassend eine in vitro gezüchtete Knorpelschicht, die mit einem Knochenersatzmaterial verbunden ist. Es wird jedoch kein lebendes Knochengewebe offenbart. WO 99/25396 beschreibt ein Mischgewebe enthaltend eine Mischung dissoziierter Chondrozyten und Osteoblasten in und auf einer polymeren Matrix. Es wird somit ein Hybridgewebe offenbart, das weder ein Knochengewebe noch ein Knorpelgewebe ist. WO 96/03160 offenbart eine Suspension enthaltend unter anderem Chondrozyten und Osteozyten und Fibrinogen, die mit einer Thrombinlösung zu einer Zell-Fibrinmatrix umgesetzt werden. EP 0 339 607 offenbart eine Zusammensetzung, die Chondrozyten oder Osteoblasten oder andere Zellen enthalten kann, in Fibrinkleber. DE 195 43 110 offenbart ein steriles Knochenmaterial nativen Ursprungs für die Transplantation, das im wesentlichen frei von Fett, Bindegewebe und Knorpelmasse ist und durch trockene Erhitzung und nachfolgende Dampfsterilisation erhältlich ist. Das Knochenmaterial kann auch in Granulatform vorliegen. Es ist bekannt, daß bestimmte Wachstumsfaktoren die Proliferation von Zellen verschiedenster Art stimulieren können. Die internationale Anmeldung WO 95/22611 beschreibt Verfahren und Zusammensetzungen zum Gentransfer in Knochenzellen in vivo, insbesondere zum Gentransfer von osteotropen Genen, die das Wachstum von Knochenvorläuferzellen in vivo stimulieren sollen.

Die WO 99/25396 offenbart ein Zellen enthaltendes Implantat, das eine polymere Matrix und eine Mischung von wenigstens zwei Zelltypen umfaßt.

Die DE 34 10 631 oder die EP 0 339 607 beschreiben ein Implantationsmaterial zur Wiederherstellung defekter Knorpel und Knochen, das entweder embryonale Chondrozyten oder Mesenchymzellen enthalten kann, wobei diese Zellen in einem biologischen Kleber enthalten sind.

Die WO 96/03160 offenbart eine Suspension von Zellen in einer fibrinogenen Lösung, die an die zu reparierende Stelle gebracht wird. Durch Zugabe von Thrombin und Calcium wird eine Fibrinmatrix gebildet, die die Zellen enthält.

Die US-PS 5,374,550 beschreibt ein Verfahren zur Entwicklung von Knorpelimplantaten, wobei Chondrozyten mit einem Chondrozyten-stimulierenden Faktor (TDCSF) behandelt werden.

Die WO 99/21497 beschreibt Instrumente und Verfahren zur Reparatur von Defekten im Gelenkknorpel, wobei im wesentlichen zylinderförmige Gewebesäulen ausgestanzt und an andere Stellen verpflanzt werden.

Bislang sind also erfolgreich lediglich Einzelkomponenten rekonstruiert worden, zum Beispiel Knochen oder Knorpel. Biologische Einzelkomponenten können komplexe, meist osteochondrale Defekte aber nicht ausreichend rekonstruieren.

Ungelöst ist nach wie vor die Rekonstruktion komplexer Gelenkstrukturen bzw. der komplette biologische Gelenkersatz mit Knochen-, Knorpel-, Kapsel- und Bandanteilen.

Eine Aufgabe der vorliegenden Erfindung ist es, ein vorteilhaftes biologisches Gelenkkonstrukt zur Verfügung zu stellen.

Diese Aufgabe wurde durch das erfindungsgemäße biologische, zumindest teilweise in vitro hergestellte Gelenkkonstrukt gelöst. Im Rahmen dieser Anmeldung bedeutet "in vitro", daß ein Prozeß außerhalb des tierischen oder menschlichen Körpers stattfindet. Dazu zählt auch die sogenannte "ex vivo"-Manipulation von Zellen, also das Kultivieren von isolierten Zellen, ihre Vermehrung und Veränderung. Es handelt sich aber nicht um ein im menschlichen oder tierischen Körper natürlich gewachsenes Gelenkkonstrukt. Das erfindungsgemäße Gelenkkonstrukt umfaßt wenigstens ein bioverträgliches Trägermaterial, Knorpelgewebe enthaltend Chondrozyten und/oder Chondroblasten und Knorpelsubstanz, Knochengewebe enthaltend Osteoblasten und/oder Osteocyten und Knochensubstanz, wobei Knorpel- und Knochengewebe fest miteinander verbunden sind.

Trägermaterialien sind Materialien, die keine zytotoxischen Effekte besitzen, die die Anhaftung von Zellen ermöglichen und die Proliferation und Differenzierung der Zellen zu gewebesynthetisierenden Zellen erlauben. Weiterhin sollte das Material einen stabilen, physiologischen pH-Wert aufweisen. Zur Analyse dieser Kriterien können folgende Untersuchungen durchgeführt werden:

Durch Elektronenmikroskopie kann die Oberflächentopographie des Materials analysiert werden und die Anhaftung der Zellen überprüft werden. Stoffwechseltests, beispielsweise der XTT-Test (cell proliferation kit, erhältlich von Roche Diagnostics, Mannheim) liefern eine Aussage über die Proliferation der Zellen durch Messung ihrer Stoffwechselaktivität. Es handelt sich hierbei um einen Proliferationstest für lebende Zellen, bei dem ein Natrium-Tetrazolium-Carboxanilit, kurz Tetrazoliumsalz (mit der Abkürzung XTT), dem Medium zugegeben wird. XTT entspricht Sodium-Tetrazolium-Carboxanilit. Damit kann ein zytotoxischer Effekt ausgeschlossen werden. Durch histologische Methoden können die gewebetypischen Matrizes von Knorpel, Knochen oder Kapsel nachgewiesen werden. Die gewebetypische Matrix kann auch durch immunhistochemische Verfahren bestimmt werden.

Für Knorpelgewebe werden als Trägersubstanz bevorzugt schwammartige Vliese, visköse, gelierende und sich verfestigende Gele oder netzartige Fadengewebe eingesetzt. Als biologische Varianten kommen dabei Fibrin-Thrombin-Komplexe, Kollagengele oder Alginate in Betracht. Als synthetische Materialien sind Kollagen, Hydrogele oder visköse Polymere denkbar. Sollen für Knorpel vliesartige Trägermaterialien verwendet werden, so können als biologische Variante Kollagenvliese und als synthetische Varianten Polylactid, Polyglycolid oder Polyurethan verwendet werden.

Die Trägermaterialien für Knochen weisen in der Regel eine feste, formbare bzw. bearbeitbare, dreidimensionale poröse Struktur auf (Porosität ca. 80-90%), wobei die Poren untereinander verbunden sein können. Die Oberflächentopographie sollte leicht gerauht sein. Trägersubstanzen können im wesentlichen aus Kollagen, Calciumphosphat oder Fibrin bestehen. Sie können jedoch auch aus synthetischen Polymeren bestehen. Als Knochen-Trägermaterialien können folgende stabile, dreidimensionale, poröse Materialien verwendet werden: humaner oder animaler spongiöser Knochen, gesinterter Knochen, Korallenmatrix, demineralisierte Knochenmatrix (biologische Varianten), Calciumphosphat-Verbindungen, Polylactid, Polyglycolid, andere Polymere (synthetische varianten). Als visköse, gelierende und sich verfestigende Gele sind folgende Materialien einsetzbar: Fibrin-Thrombin-Komplexe, Kollagengele, Alginate (biologische Varianten), Hydrogele, visköse Polymere (synthetische Varianten). Die Knochen-Trägermaterialien können weiterhin mit Haftmolekülen beschichtet sein, die die Anheftung von Zellen erleichtern. Solche Haftmoleküle sind bevorzugt Fibronektin oder Laminin. Wesentlich ist, daß keine Abstoßungsreaktionen gegen die Trägermaterialien in dem Empfängerorganismus auftreten.

Für Kapselkomponenten sind bandförmige Gewebenetze oder Vliese geeignet. Als Trägermaterialien zur Herstellung von Bandkomponenten sind allgemein membranartige synthetische resorbierbare Fasermaterialien einsetzbar, beispielsweise Polyglactin, Polyglycolsäure, Polymilchsäure oder Hyaluronsäure.

Die Trägermaterialien für Bandkomponenten können auch als Trägermaterialien für Kapselkomponenten verwendet werden.

Ein weiterer Aspekt der vorliegenden Erfindung ist die Verwendung von Spongiosa als Trägermaterial der Knochenkomponente. Überraschenderweise wurde gefunden, daß Spongiosa sehr gut geeignet ist, um Knochenvorläuferzellen zur Synthese von Knochensubstanz anzuregen. Vorzugsweise handelt es sich dabei um autoklavierte, humane, allogene Spongiosa, die aus Hüftköpfen gewonnen wird. Am bevorzugtesten wird das Material aus Hüftköpfen gewonnen, die durch Wasserstrahl und Ultraschallbehandlung von losem Stroma gereinigt werden. Anschließend erfolgt Autoklavierung bei 134°C und 2,5 bar zur Sterilisation. Das Material ist steril, nicht immunogen, resorbierbar und besteht aus Hydroxylapatit und denaturiertem Kollagen Typ 1.

Das in dem erfindungsgemäßen Gelenkkonstrukt enthaltene Knorpelgewebe enthält Chondrozyten und/oder Chondroblasten und Knorpelsubstanz. Unter Knorpelgewebe ist ein aus Knorpelzellen (Chondrozyten) und - verschiedener - Grundsubstanz und verschiedenen Faserarten bestehendes viskoelastisches, gefäßund nervenloses Stützgewebe zu verstehen. Unter Knorpelsubstanz ist die Grundsubstanz zusammen mit den verschiedenen Faserarten zu verstehen. Die Grundsubstanz enthält Glycosaminoglykane und Proteoglykane, vorzugsweise Hyaluronsäure, Chondroitinsulfat und Keratansulfat. Weiter sind Proteine und Mineralbestandteile enthalten. Als Faserarten können elastische Fasern, kollagene Fibrillen oder kollagene Fasern vorkommen.

Unter Knochengewebe ist ein Gewebe zu verstehen, das aus Knochenzellen, kollagenen Fasern und einer verkalkten Grundsubstanz besteht. Knochensubstanz im Sinne der Anmeldung umfaßt Kollagen, Glycosaminglykane und Proteoglykane, sowie anorganische Substanzen, hauptsächlich Calciumphosphat, das in Form von Hydroxylapatit-Kristallen auftritt. Zur Verbesserung der Gefäßneubildung kann das Knochengewebe Wachstumsfaktorproteine enthalten oder Osteoblasten, die mit für Wachstumsfaktoren kodierenden Genen transfiziert wurden. Schließlich können auch gefäßbildende Zellen enthalten sein, die die Blutgefäßentstehung fördern (z.B. Endothelzellen oder deren Vorläuferzellen).

Erfindungsgemäß sind Knorpelgewebe und Knochengewebe des Gelenkkonstrukts fest miteinander verbunden. Dabei ist das Trägermaterial des Knochens mit der neuen Knochensubstanz verzahnt, es ist auch teilweise in die Knorpelkomponente integriert. Es liegt also keine scharfe Trennfläche zwischen Knochen- und Knorpelgewebe vor, vielmehr wird durch Verzahnung und Interdigitation eine feste Verbindung erreicht. Bevorzugt wird dies dadurch erreicht, daß das Knochengewebe eine rauhe bzw. poröse Oberfläche aufweist, in die das Knorpelgewebe einwachsen kann.

Die in dem Gelenkkonstrukt enthaltenen Knochen- und Knorpelzellen sind vor der Herstellung des Gelenkkonstrukts nach üblichen Methoden isoliert worden. Es können auch Vorläuferzellen isoliert werden, die erst während Kultur in vitro zu Knochen- oder Knorpelzellen differenzieren.

Das biologische Gelenkkonstrukt kann beliebige Größe aufweisen. Damit ist der Teilersatz einer Gelenkfläche, der Totalersatz einer Gelenkfläche oder der Ersatz eines gesamten Gelenks mit zwei Gelenkflächen möglich.

Beim Teilersatz einer Gelenkfläche (ohne Bandersatz) kann ein einzelnes präformiertes Konstrukt oder osteochondrale Zylinder verwendet werden. Bei Verwendung eines einzelnen präformierten Konstrukts wird ein bestimmter Knorpel/Knochendefekt eines Gelenks durch ein einzelnes osteochondrales Konstrukt ersetzt. Die Form wird dabei durch die Knochenkomponente bestimmt. Nach Zellgewinnung und Herstellung der Komponenten wird das Konstrukt ex vivo präformiert, das heißt es wird anhand individueller Datenerhebung durch Computertomographie oder Magnetresonanzuntersuchungen auf den zu korrigierenden Defekt hin maßgeschneidert. Die Verankerung erfolgt dann durch den Knochenzapfen. Zur Vermeidung einer jeweiligen individuellen Anfertigung eines Konstrukts wie beschrieben können auch uniforme osteochondrale Zylinder angefertigt werden, die im Sinne einer sogenannten Mosaikplastik zu mehreren in einen Defekt eingefügt werden und die Gelenkfläche rekonstruieren. Diese Zylinder können in verschiedenen Größen und Formen angefertigt werden. In jedem Fall bestehen sie aus einer Knorpelschicht und einem Knochenzapfen. Beide Komponenten sind mit der jeweiligen Zellsorte beladen. Die Knochenzellen können auch mit Wachstumsfaktoren transfiziert sein, um zusätzlich die Knochenbildung und Gefäßbildung einzuleiten. Vorzugsweise haben die Zylinder eine Höhe von 5 bis 25 mm und einen Durchmesser von 2,5 bis 15 mm. Die Schichtdicke des Knorpels beträgt vorzugsweise 0,5 bis 2 mm, die des Knochens wird üblicherweise zwischen 3,5 und 23 mm variieren. Als mögliche Formen sind Zylinder mit rundem Querschnitt, mit dreieckigem Querschnitt oder mit mehreckigem Querschnitt (z.B. penta-, hexa-, hepta- oder oktogonal) denkbar. Ebenfalls können Quader mit rechteckigem Querschnitt (viereckig) bzw. mit quadratischem Querschnitt verwendet werden. Die Knorpel sind so geformt, daß ihre Oberfläche eine konvexe Krümmung aufweist oder eine plane Oberfläche ohne Krümmung ist. Die Verwendung osteochondraler Zylinder wurde im Kaninchenmodell überprüft und war erfolgreich in der Rekonstruktion eines osteochondralen Defekts. Der Vorteil der Methode ist die einfachere Herstellung und die mögliche minimal-invasive Anwendung.

Beim Totalersatz einer Gelenkfläche weist das Gelenkkonstrukt vorzugsweise eine anatomisch sinnvolle Form auf, beispielsweise besitzt es eine Gelenkseite, deren Oberfläche aus Knorpelgewebe besteht und die Kontakt zu einem anderen Gelenkteil haben kann, und es besitzt eine Ankerseite, die zum Teil aus Knochengewebe besteht und die zur Verankerung des Konstrukts in einem Knochenschaft dienen kann. Die Ankerseite ist vorzugsweise in der Form eines zylinderförmigen Zapfens ausgebildet, der besonders gut in einem Knochenschaft verankert werden kann. Die Gelenkseite besitzt besonders bevorzugt eine konkave oder konvexe Oberfläche. Es sind aber auch andere Gelenkkonstruktformen denkbar, je nach Art des Gelenks, für dessen Reparatur das Konstrukt verwendet werden soll.

Die erfindungsgemäßen Gelenkkonstrukte können ebenfalls wenigstens eine Bandkomponente aufweisen. Die Bandkomponente besteht beispielsweise aus einem strangförmigen, faserartigen oder membranösen Biomaterial. Sie kann an Bandverbindungsstellen der Knochenkomponente des erfindungsgemäßen Gelenkkonstrukts befestigt werden. Mögliche Befestigungsarten sind biologische Klebung, beispielsweise durch Fibrin-Thrombin-Komplexe, unterstützende, transossäre Naht oder der Durchzug durch die Knochenkomponente in einem Knochenkanal. Beim Totalersatz einer Gelenkfläche ist die Anfertigung von Bandstrukturen nicht unbedingt notwendig, da die vorhandenen Kapsel-Bandstrukturen im Defekt genutzt werden können. Die Knochenkomponente kann Verankerungspunkte für diese Strukturen aufweisen. Dies sind in der Regel Bohrkanäle, durch die Nahtmaterial hindurchgezogen und damit die Kapsel-Bandstrukturen fixiert werden können. Vorzugsweise wird das Knorpel-Knochenkonstrukt ohne zusätzliche Fixierung der Kapsel oder Bänder eingesetzt.

Eine weitere Ausführungsform der vorliegenden Erfindung besteht in einem biologischen Gelenkersatz, der wenigstens zwei Gelenkkonstrukte umfaßt. Zwei Gelenkkonstrukte können dabei mit Ihren knorpelbeschichteten Gelenkseiten Kontakt zueinander haben, während die voneinander abgewandten Ankerseiten in zwei verschiedenen Knochenschäften verankert werden können. Dieser Gelenkersatz wird vorzugsweise weiter durch Anbringung von wenigstens zwei Bandkomponenten stabilisiert. Schließlich kann ein derartiger biologischer Gelenkersatz auch eine Gelenkkapsel aufweisen. Dazu wird ein membranöses Biomaterial an Kapselverbindungsarealen der Knochenkomponente entweder durch biologische Klebung oder durch Naht befestigt. Durch die beschriebene Ausführungsform kann der komplette Ersatz eines Gelenks mit zwei Gelenkflächen erfolgen. Der komplette Gelenkersatz mit zwei Gelenkanteilen ist vor allem bei Arthrose oder Arthritis notwendig und stellt die komplizierteste Form des Gelenkersatzes dar. Prinzipiell werden zwei Gelenkteile bestehend aus Knorpel- und Knochenkomponente hergestellt. Auf einer Seite ist die Gelenkfläche konvex, auf der anderen passend dazu konkav geformt, so daß eine regelrechte Artikulation der Flächen bei Bewegung möglich ist. Die Komponenten werden einzeln hergestellt und können auch einzeln implantiert werden, wie der Totalersatz nur einer Gelenkfläche wie oben stehend beschrieben.

Bevorzugt werden vorhandene Kapseln und Bänder des alten Gelenks benutzt, um die Einzelteile zu verbinden und das neue Gelenk zu stabilisieren. Dazu werden die Bänder durch Nähte, welche durch Bohrkanäle gezogen werden, an der Knochenkomponente fixiert. Die Komponente kann an dieser Stelle eine Vertiefung aufweisen. Alternativ werden die beiden Gelenkteile durch Nahtmaterial miteinander verbunden, bis sich nach der Transplantation eine Neokapsel in vivo gebildet hat. Sollte ein Bandersatz durch Tissue engineering notwendig sein, so kann der Bandersatz wie folgt hergestellt werden. Zunächst werden autologe Fibroblasten aus der Dermis durch eine Hautbiopsie isoliert und in vitro vermehrt. Ein bandförmiges Biomaterial wird mit einer Zellsuspension (2,5 bis 5 x 10⁷ Zellen pro ml Medium) besiedelt. Als Biomaterial kommen beispielsweise Collagenvliese, azelluläre Lederhaut, lyophilisierte Dura oder synthetische Bänder aus PGLA in Betracht. Das Bandkonstrukt wird für 3 bis 7 Tage in vitro gezüchtet. Schließlich wird die Struktur an der Knochenkomponente durch Naht oder Fibrinklebung befestigt. Dabei erfolgt die Fixierung zunächst nur auf einer Gelenkseite, die zweite Verbindung erfolgt erst nach Implantation im Rahmen der Operation.

Alternativ können die Bandstrukturen auch durch die Verpflanzung von Sehnen oder deren Teilen erfolgen, wie es bereits gegenwärtiger Stand der chirurgischen Technik ist.

Die vorliegende Erfindung betrifft außerdem ein Verfahren zur Herstellung eines biologischen Gelenkkonstrukts. Dieses Verfahren umfaßt die Bereitstellung einer Knochenkomponente, die Bereitstellung einer Knorpelkomponente, die Verbindung dieser beiden Komponenten sowie die Züchtung des resultierenden Konstrukts in vitro.

Die Bereitstellung der Knochenkomponente erfolgt durch Besiedelung eines bioverträglichen Trägermaterials mit Osteoblasten. Als Trägermaterialien kommen die obengenannten Trägermaterialien für Knochen in Betracht. Vorläuferzellen von Osteoblasten können durch kleine Knochenbiopsien des Beckens, Brustbeines, des Schädels und Kiefers oder von Röhrenknochen gewonnen werden. Aus den Knochenproben wird das lose Stroma herausgespült und nach Zentrifugation in einer Kulturflasche ausplattiert. Die festen Knochenanteile können ebenfalls in Kultur gebracht werden, da hieraus durch Migration weitere Zellen gewonnen werden können. Die auswachsenden Zellen werden im subkonfluenten Stadium gesplittet und zwei- bis dreimal passagiert. Alternativ zu Knochenproben können auch Aspirate von Knochenmark aus dem Becken oder aus dem Brustbein verwendet werden. Hierbei wird der Knochen beispielsweise mit einer Kanüle punktiert, und die Probe wird durch Ansaugen (Aspiration) erhalten. Die Aspirate werden in Heparinmedium gespült, durch einen Dichtegradienten zentrifugiert, um rote Blutkörperchen abzutrennen und schließlich in einer Kulturflasche ausplattiert. Der osteoblastische Phänotyp der Zellen in Kultur kann durch Nachweis der knochenspezifischen Proteine Alkalische Phosphatase und Osteocalcin im Kulturmedium überprüft werden. Immunhistochemische Färbungen von Kontrollkulturen sind ebenfalls denkbar. Die Blutversorgung des biologischen Gelenkersatzes durch neugebildete Gefäße ist von entscheidender Bedeutung für das Überleben der Zellen und das Einheilen des Konstrukts. Aus diesem Grund kann die Knochenkomponente auf drei Weisen modifiziert werden: Sie kann Wachstumsfaktorproteine enthalten, die die Gefäßneubildung induzieren (1), sie kann Osteoblasten enthalten, die mit Wachstumsfaktorgenen transfiziert sind (2) oder sie kann gefäßbildende Zellen enthalten, die zur Blutgefäßentstehung führen (3).

Angiogene Wachstumsfaktoren können direkt in rekombinanter Form in der viskösen Matrix der Knochenkomponente in wirksamer Konzentration gespeichert werden. Mögliche Faktoren sind Vascular endothelial growth factor (VEGF) sowie Isoformen davon, Basic fibroblast growth factor (bFGF), Angiopoetin 1 und 2 (Ang I und II). Die Konzentration der Faktoren beträgt üblicherweise 0,1 bis 10 ng/ml visköse Matrix. Der oder die Wachstumsfaktoren können vorteilhaft einer Calciumchlorid-Thrombinlösung zugesetzt werden, die dann zu einer Osteoblastensuspension in Fibrinogenlösung zugegeben wird. Dadurch bildet sich ein dreidimensionales Fibrinnetzwerk mit haftenden Osteoblasten im Kulturmedium und gespeichertem Wachstumsfaktor.

Die Transfektion von Osteoblasten mit gefäßbildenden Wachstumsfaktoren ist ausführlich in einem späteren Teil des Anmeldetextes beschrieben.

Es können auch gefäßbildende Zellen, sogenannte Endothelzellen oder deren Vorläuferzellen, in die visköse Matrix der Knochenkomponente eingebracht werden, gemischt mit den Osteoblasten. Mikrovaskuläre Endothelzellen werden aus der Lederhaut (Dermis) eines Patienten isoliert nach einem an sich bekannten Protokoll und mit Endothelzellenmedium in vitro vermehrt (etablierte Technik). Eine Osteoblasten-Fibrinogensuspension wird hergestellt, wobei als Medium sogenanntes Endothelzellmedium verwendet wird, in welchem Osteoblasten gut wachsen können. Die gemischtzellige Osteoblasten-Endothelzell-Fibrinogensuspension wird dann in die poröse, feste Trägermatrix der Knochenkomponente eingesogen bzw. appliziert.

Die drei beschriebenen Strategien können auch kombiniert werden. Beispielsweise kann eine Osteoblasten-Endothelzell-Fibrinogensuspension eingesetzt werden zusammen mit Osteoblasten, die mit Wachstumsfaktorgenen transfiziert wurden. Genauso können Osteoblasten-Endothelzell-Fibrinogensuspensionen gemeinsam mit rekombinanten Wachstumsfaktoren verwendet werden.

Vorzugsweise wird das Knochenträgermaterial durch eine Knochenzellsuspension in einer Aspirationskammer besiedelt. Üblicherweise wird das Knochenträgermaterial vor der Besiedelung durch Knochenzellen in anatomisch sinnvoller Weise geformt. Beispielsweise führt die Formgebung zur Ausbildung einer Gelenkseite, die zur Aufnahme einer Knorpelfläche dienen soll und zur Ausbildung einer Ankerseite, die der Verbindung mit einem Knochenschaft dienen soll.

Chondrozyten werden bevorzugt aus Biopsien von Knorpelgewebe des Ohres, der Rippe und der Gelenke durch enzymatische Auflösung gewonnen. Durch Beimpfung von Biomaterialien wie Kollagenschwämmen und Fibrinkleber können die Zellen organotypisch in einer dreidimensionalen Kultur weiter vermehrt werden. Dadurch wird auch vermieden, daß sich die Zellen zu Fibroblasten entdifferenzieren. Der chondroblastische Phänotyp bleibt erhalten. Zur Bereitstellung der Knorpelkomponente kann nun eine Suspension von Chondrozyten hergestellt werden entweder in einem flüssigen Medium oder in einem gelartigen Material. Beispielsweise können Knorpelzellen in der Thrombinkomponente eines Fibrinklebers suspendiert werden und nach Mischen mit der Fibrinogenkomponente in eine bestimmte vorgefertigte Kulturform gegossen werden. Dadurch entsteht ein festes Gebilde, das mit Kulturmedium überschichtet wird und im Brutschrank gezüchtet wird. Alternativ können auch bioverträgliche Trägersubstanzen durch Knorpelzellen besiedelt werden. Beispielsweise werden Schwämme aus Kollagen mit Chondrozyten angeimpft. Die angeimpften Kollagenvliese können im Brutschrank kultiviert werden. In einer bevorzugten Ausführungsform der Erfindung werden Knochen- und Knorpelkomponente getrennt voneinander hergestellt und getrennt kultiviert. Nachdem die jeweiligen Zellen ausreichend Gewebe gebildet haben, werden Knochen- und Knorpelkomponente durch Fibrinklebung miteinander verbunden. Es ist wesentlich für die Erfindung, daß bei der Verbindung von Knochen- und Knorpelkomponente das Trägermaterial der Knochenkomponente in den Knorpel integriert wird. Das resultierende Konstrukt kann nun in vitro kultiviert werden, so daß die Zellen zur Anheftung und zur Synthese ihrer gewebespezifischen extrazellulären Matrix stimuliert werden, dadurch wird eine biologische Vernetzung der zusammengesetzten Komponenten erreicht.

In einer besonders bevorzugten Ausführungsform der Erfindung werden Knochen- und Knorpelkomponente nicht separat hergestellt und kultiviert. Vielmehr wird während des Abbindens des Fibrinklebers bei der Herstellung der Knorpelkomponente das Trägermaterial der Knochenkomponente, das noch nicht durch Osteoblasten besiedelt worden ist, in die Knorpelschicht gedrückt, so daß es fest eingebunden wird. Die Besiedelung durch Osteoblasten erfolgt erst später.

Eine weitere Ausführungsform der Erfindung umfaßt auch die Herstellung einer Bandkomponente aus faserartigen Materialien und Fibroblasten. Dabei können Fibroblasten aus Suspension auf bandförmige Materialien geimpft werden und im Brutschrank kultiviert werden. Ähnlich dazu können Ausführungsformen die Herstellung auch einer Kapselkomponente aus faserartigen, membranösen Materialien und Fibroblasten umfassen.

Weiterhin kann das erfindungsgemäße Verfahren das Anbringen von Bandverbindungsstellen oder Kapselverbindungsarealen an dem Trägermaterial der Knochenkomponente umfassen. Daran können Gelenkbänder bzw. Kapselkomponenten befestigt werden. Bandkonstrukte werden bevorzugt dadurch mit der Knochenkomponente verbunden, daß das Bandkonstrukt durch einen Bohrkanal in der Knochenkomponente hindurchgezogen wird.

Ein weiterer Aspekt der Erfindung ist ein Verfahren zur Herstellung von Knochengewebe, wonach Knochen- oder Knochenvorläuferzellen isoliert werden, diese Zellen durch nicht-viralen Gentransfer mit einem Wachstumsfaktorgen transfiziert werden und zur Besiedelung eines bioverträglichen Trägermaterials benutzt werden. Das entstehende Konstrukt wird schließlich in vitro weitergezüchtet. Gegebenenfalls werden die isolierten Zellen vor der Transfektion in vitro vermehrt. Die Transfektion kann stabil sein, bevorzugt ist jedoch eine transiente Transfektion. Bei der transienten Transfektion werden die Zellen nur vorübergehend, also nicht dauerhaft durch die Einschleusung von DNA genetisch verändert. Die Transfektion kann nach verschiedenen Methoden erfolgen. Beim Partikel-vermittelten Gentransfer werden Zellen durch eine sogenannte Genkanone (gene gun) mit Plasmid- oder nackter DNAbeschichteten Goldpartikeln beschossen. Alternativ dazu kann die Transfektion durch Elektroporation vorgenommen werden. Die bevorzugte Transfektionsmethode ist jedoch die Lipofektion. Bevorzugt werden dabei die Reagenzien Transfectam® (erhältlich von der Firma Promega), Fugene® (erhältlich von der Firma Roche Diagnostics), Lipofectamin 2000®, Lipofectin® (Firma Life Technology) und Escort® (erhältlich von der Firma Sigma) verwendet.

Erfindungsgemäß werden die Zellen mit wenigstens einem Wachstumsfaktorgen transfiziert. Ein Wachstumsfaktor oder Cytokin im Sinne der Erfindung ist eine Verbindung, die die Proliferation und/oder Differenzierung von Zellen stimulieren kann. Die meisten Wachstumsfaktoren sind Proteine oder Peptide, so daß Gene, die für diese Proteine oder Peptide kodieren, transfiziert werden können. Bevorzugt sind diese Gene in Plasmide eingebaut, die zusätzliche regulatorische und Kontrollsequenzen enthalten, um die Expression des Gens zu gewährleisten. Zu diesen zusätzlichen Sequenzen zählen ein Replikationsursprung, eine Promotorsequenz, am bevorzugtesten abgeleitet vom Cytomegalievirus (CMV), gegebenenfalls eine Kozaksequenz (eine nahe am Promotor lokalisierte DNA-Sequenz, die die Expression erhöhen soll), ein Polyadenylierungssignal, gegebenenfalls ein Gen, das Resistenz gegen ein Antibiotikum vermittelt. Gemäß der Erfindung können auch Nucleinsäuren transfiziert werden, die nur für ein Fragment eines Wachstumsfaktors kodieren, das aber die Aktivität eines Wachstumsfaktors besitzt.

Mögliche Wachstumsfaktoren, deren Gene transfiziert werden können sind "basic fibroblast growth factor" (bFGF), "insuline-like growth factor" (IGF I, II), "platelet derived growth factor" (PDGF-AA, -AB, -BB), "bone morphogenetic proteins" (BMP-1 bis BMP-20), "vascular endothelial growth factor" (VEGF), Faktor XIII (F XIII), "transforming growth factor β" (TGF-β), Angiopoetin (Ang I, II) und andere osteotrope Faktoren. Bevorzugt wird jedoch das Gen transfiziert, das für epidermalen Wachstumsfaktor kodiert (EGF).

In einer besonderen Ausführungsform der Erfindung wird das bioverträgliche Trägermaterial nicht nur mit transfizierten Zellen, sondern auch mit nicht transfizierten Zellen in einem Verhältnis von 1:3 bis 1:9 (transfizierte zu nicht-transfizierten Zellen) besiedelt. Die isolierten Zellen können verschiede Knochenzellen oder Knochenvorläuferzellen sein, bevorzugt sind es jedoch stromale Zellen.

Außerdem betrifft die Erfindung Knochengewebe, das wenigstens ein bioverträgliches Trägermaterial und Osteoblasten enthält, die in vitro durch nicht-viralen Gentransfer mit einem Gen, das für einen Wachstumsfaktor kodiert, transfiziert worden sind. Bevorzugte Ausführungsformen betreffen Knochengewebe, die nach einem der oben beschriebenen Verfahren zur Herstellung von Knochengewebe mit Hilfe von transfizierten Zellen hergestellt wurden.

Das erfindungsgemäße Knochengewebe hat zwei wesentliche Vorteile.

Zum Einen werden nicht-transfizierte Zellen durch die sezernierten Wachstumsfaktoren parakrin zur Proliferation angeregt. Dabei wird der Wachstumsfaktor von einer Zelle produziert und wirkt auf eine andere Zelle ein. Das hat nicht nur eine verbesserte Besiedelung der Trägermaterialien während der in vitro-Phase zur Folge, sondern auch eine verbesserte Einbindung des Gewebes nach Implantation. Das Knochengewebe stellt dann in vivo Wachstumsfaktoren zur Verfügung, ist also ein "zelluläres Wachstumsfaktor-Liefersystem". Dabei treten die von anderen Verfahren bekannten Nachteile nicht auf: Es werden keine Zellen außer den gewünschten transfiziert. Es sind keine Risiken zu befürchten, die mit viralen Methoden einhergehen. Es müssen nicht wiederholt Dosen von rekombinantem Wachstumsfaktorprotein appliziert werden, da die Sekretion "physiologisch" erfolgt. Bei direkter Gabe von Wachstumsfaktor bestehen auch die Risiken biologischer Inaktivität oder mangelnder Reinheit des Proteins.

Der zweite wesentliche Vorteil besteht darin, daß bestimmte Wachstumsfaktoren nach Implantation des Gewebes eine Gefäßneubildung induzieren können. Die Gefäßneubildung ist eines der entscheidenden Probleme beim "TissueEngineering". Die Faktoren können somit zu einer verbesserten Vaskularisierung des Konstrukts führen, was ein wesentlicher Vorteil für die Eigenschaften des implantierten Gewebes ist.

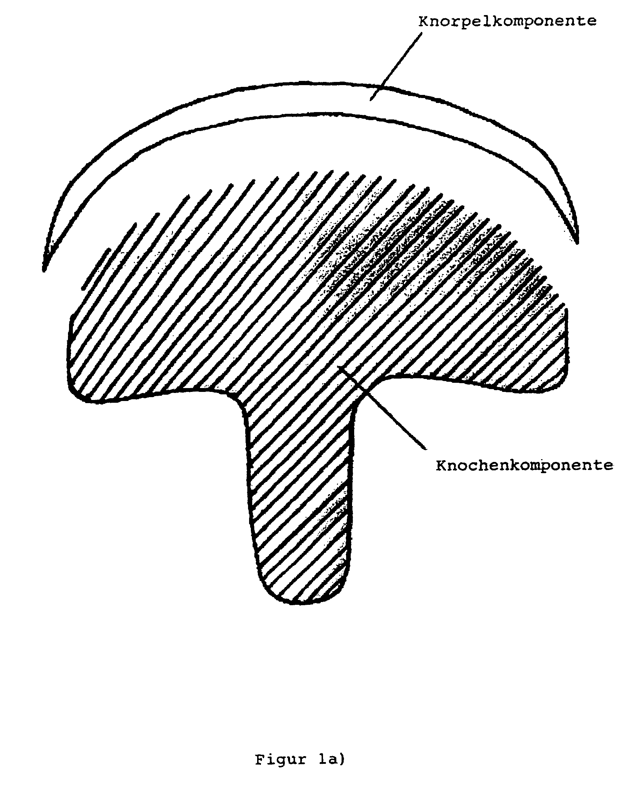

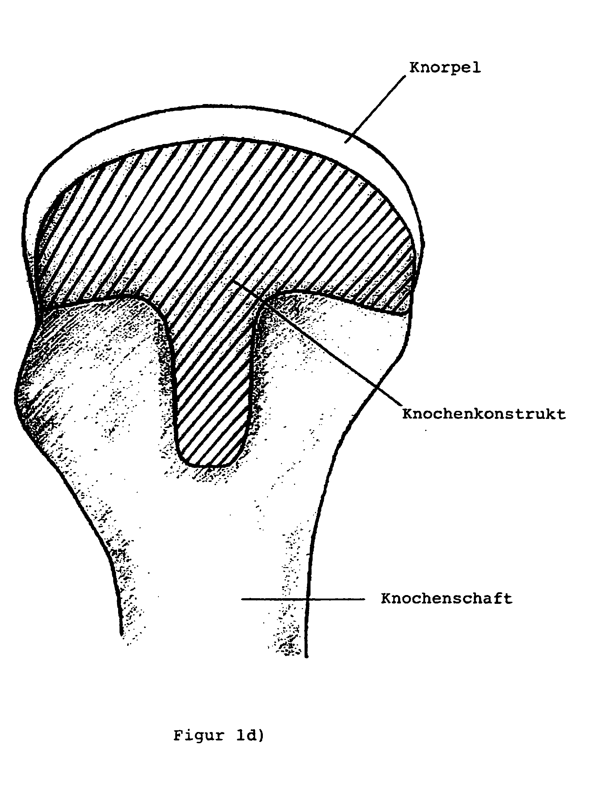

Figur 1a) zeigt die Fusion der Knorpelkomponente mit dem Trägermaterial des Knochens. Während dem Verfestigungsvorgang der Knorpelkomponente wird das Trägermaterial der Knochenkomponente in das Knorpelkonstrukt eingedrückt.

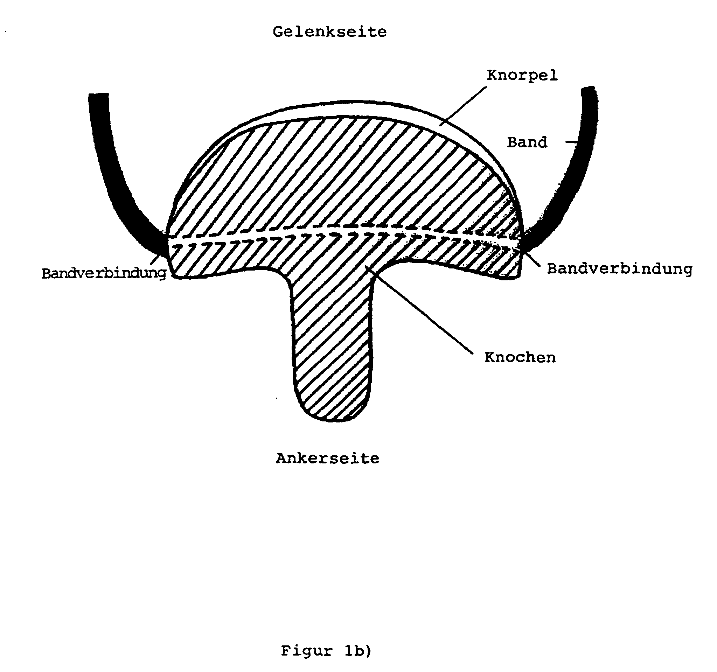

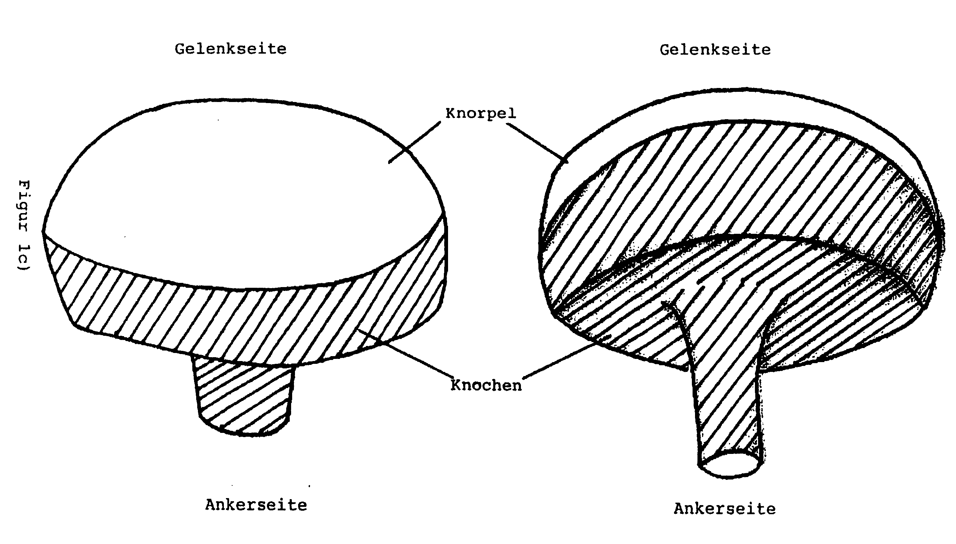

Figur 1b) zeigt das Anbringen der Bandkomponenten. Biologische Bandkomponenten können an den Bandverbindungsstellen integriert werden (hier durch transossären Durchzug). Das Konstrukt hat eine Gelenkseite und eine Ankerseite für den Einbau in den Knochen.

Figur 1c) zeigt Ansichten eines Knorpel-Knochen-Konstrukts. Das Trägermaterial der Knochenkomponente wurde mit Osteoblasten besiedelt. Die Knorpelkomponente ist fest mit der Knochenkomponente Verbunden. Das Konstrukt hat eine anatomisch korrekte Form zum Gelenkersatz. Es weist eine Gelenkseite und eine Ankerseite auf.

Figur 1d) zeigt die Integration des biologischen Gelenkkonstrukts in den Knochen. Darstellung eines einseitigen Gelenkersatzes durch Einbau eines Knorpel-Knochen-Konstrukts ohne Bandverbindung in den Knochen.

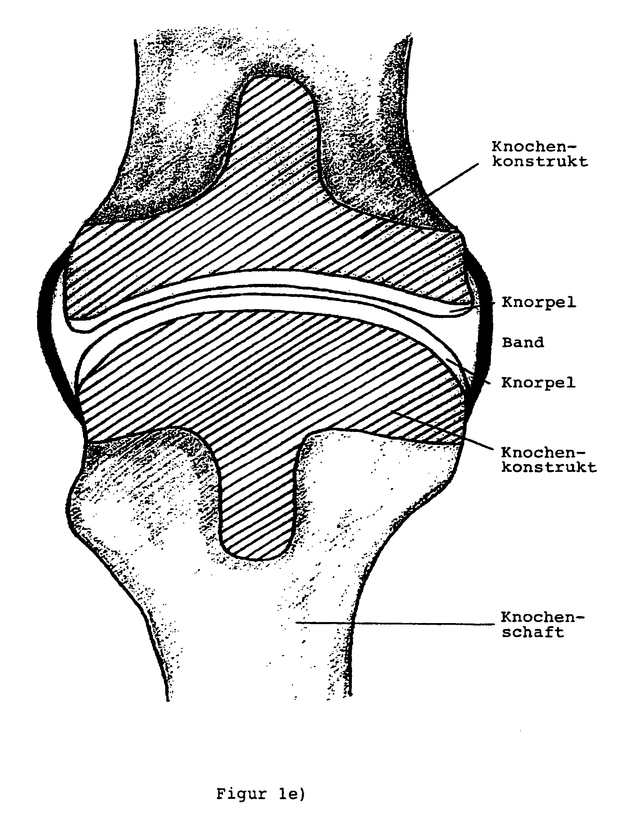

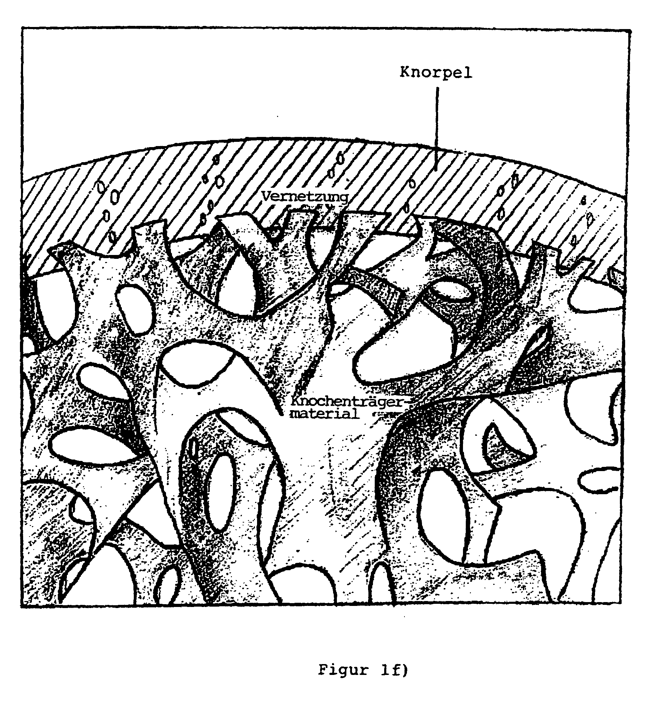

Figur 1e) zeigt die Integration eines kompletten Gelenkersatzes mit Bandverbindung. Ein zweiteiliger Gelenkersatz wurde mit Bandverbindung in Knochen nach Entfernung des ursprünglichen Gelenks (hier Fingergrundgelenk) eingesetzt. Der biologische Gelenkersatz hat die korrekte anatomische Form.

Figur 1f) zeigt ein histologisches Schema nach Fusion der Knorpelkomponente mit dem Knochenträgermaterial. Das Trägermaterial der Knochenkomponente (hier Spongiosa) wurde während dem Verfestigungsvorgang der Knorpelzellsuspension (hier Fibrinkleber) in das Knorpelkonstrukt integriert. Es entsteht eine stabile verbindung zwischen Knorpelschicht und Trägermaterial des Knochens.

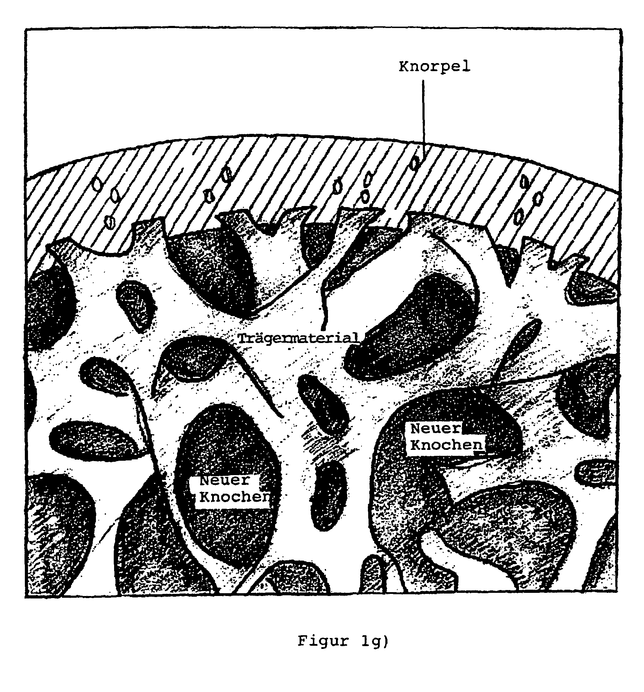

Figur 1g) zeigt ein histologisches Schema eines Knorpel-Knochen-Konstrukts nach Synthese von Knochensubstanz durch Osteoblasten. Nach Besiedelung des in Figur 1f) gezeigten Konstrukts mit Osteoblasten wurde neue Knochensubstanz synthetisiert.

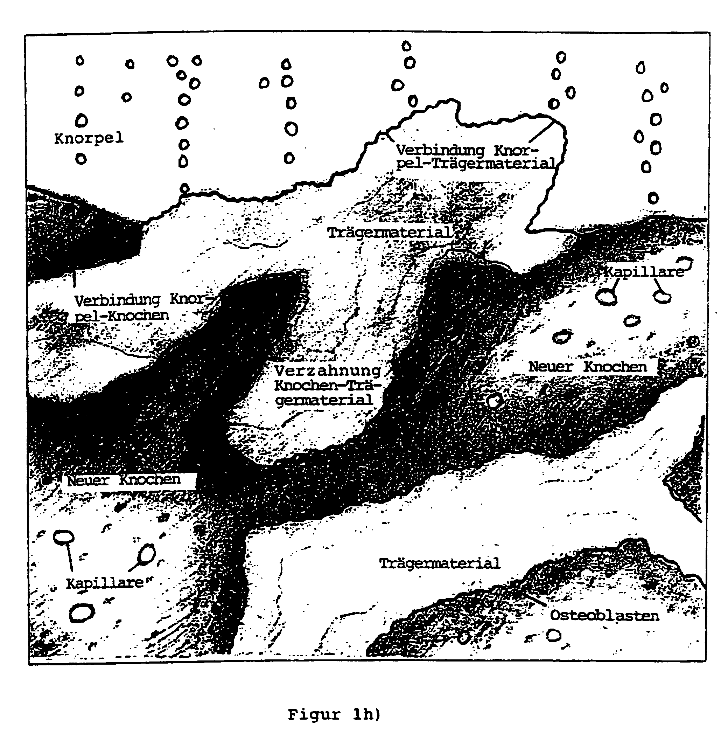

Figur 1h) zeigt ein histologisches Schema der Grenzfläche zwischen Knorpel und Knochen. Die Vergrößerung zeigt im Schema die Grenzfläche zwischen der Knorpel- und Knochenkomponente mit dem Trägermaterial des Knochens. Es zeigt sich die Verzahnung des Trägermaterials mit der neuen Knochensubstanz und die Vernetzung des Knorpels mit dem Knochen. Das Trägermaterial ist teilweise in die Knorpelkomponente integriert.

Die Erfindung wird durch nachfolgende Beispiele näher erläutert, wobei Tierversuche die Durchführbarkeit der vorliegenden Erfindung belegen müssen, da Experimente am Menschen aus ethischen Gründen nicht vertretbar erscheinen. Gegenstand der vorliegenden Erfindung sind aber bevorzugt biologische Gelenkkonstrukte, die autologe, also vom Empfänger stammende oder allogene, das heißt von einem anderen Menschen stammende Zellen enthalten.

### Beispiel 1: Isolierung von Chondroblasten

Es wird zunächst die Isolierung von Chondroblasten aus Kaninchen beschrieben. Diese Methode ist im Prinzip auf Menschen übertragbar.

Das Kaninchen wird durch eine Injektion narkotisiert. Die Haare werden im Operationsgebiet rasiert, die Haut desinfiziert und steril abgedeckt. Über dem knorpeligen Anteil der Rippe wird ein Schnitt von ca. 2 cm gesetzt. Der Rippenanteil wird freipräpariert, etwa 1,5 cm Rippenknorpel einschließlich des Perichondrium werden herausgetrennt. Nach Blutstillung und Spülung wird die Wunde durch Rückstichnähte verschlossen. Anschließend wird ein Sprühverband angebracht.

Der autologe Rippenknorpel wird in sterile Ringerlösung (DAB 7-Lösung, erhältlich von Fa. Delta Pharma) gegeben, die mit 200 IU/ml Penicillin und 20 µg/ml Streptomycin supplementiert worden ist. Der Knorpel kann in der Lösung für einige Stunden bei 4°C aufbewahrt werden, muß aber innerhalb von 6-8 Stunden bearbeitet werden. Das Knorpelgewebe wird mit einem Skalpell (20/22) unter sterilen Bedingungen auf eine Größe von 1-2 mm zerkleinert und in eine große Petrischale (130 × 15 mm) gegeben. Die Knorpelmenge wird gewogen.

Anschließend werden 50 ml 2 mg/ml Kollagenase in einem handelsüblichen, speziell für Chondrozyten hergestellten Standardmedium, nämlich HAM's F12-Medium (Hepes-Modifikation, mit 100 IU/ml Penicillin, 100 µg/ml Streptomycin) in die Petrischale mit den Knorpelstückchen gegeben. Es folgt für 16-24 h Inkubation im Zellkultur-Brutschrank.

Die Knorpelzellsuspension mit verbliebenen Knorpelstückchen wird abpipettiert, mit dem gleichen Volumen HAM's F12-Medium (Hepes-Modifikation), 100 IU/ml Penicillin, 100 µg/ml Streptomycin, ≈50 µg/ml Ascorbinsäure, ≈10% (v/v) autologes Serum, 2 mM Glutamin verdünnt und ausgiebig auf einem Schüttler gerüttelt, um die angedauten Knorpelstücke zu trennen. Dieser Schritt wird am besten in verschlossenen 50 ml-Zentrifugen-Schraubgefäßen durchgeführt.

Die Zellsuspension wird durch Zellfilter in 50 ml-Schraubgefäße filtriert (ca. 25 ml pro Gefäß) und mit o. g. HAM's F12-Medium auf 50 ml aufgefüllt. Die Zellen werden 3-4 mal mit 30 ml PBS durch Zentrifugation (10 min, 500 g, 21°C) gewaschen. Nach dem letzten Waschschritt werden die Zellen in 30 ml Medium aufgenommen.

Um die Zellzahl zu bestimmen, werden zunächst 0,5 ml einer 0,4% Trypanblaulösung in ein Teströhrchen gegeben. Nach Hinzufügen von 0,3 ml HBSS (Hanks Salzlösung) und 0,2 ml Zellsuspension wird sorgfältig gemischt und 15 Minuten bei Raumtemperatur inkubiert. Anschließend wird durch Zählung mit einem Hämozytometer die Zellzahl ermittelt.

### Beispiel 2: Isolierung von Osteoblasten

Es stehen verschiedene Methoden zur Verfügung, erstens die offene Knochenbiopsie (als Migrations- oder stromale Zellkultur) und zweitens die Aspiration von Knochenmark.

### a) Knochenmarkbiopsie

Nach örtlicher Betäubung und einem kleinen Hautschnitt wird eine sterile Knochenbiopsie durch einen Hohlbohrer entnommen. Es werden Spongiosablöckchen von 0,5 bis 1 cm³ herausgebohrt. Anschließend wird die Wunde verschlossen. Alternativ dazu können auch 15 ml Knochenmark aspiriert werden. Die Spongiosa sollte sehr schnell weiterverarbeitet werden, wenn möglich nicht länger als 12 Stunden im Transportgefäß bei 4°C lagern. Das Medium wird verworfen, die Spongiosa in die Petrischale gegeben und dort in 2 bis 3 mm kleine Partikel ("Chips") zerkleinert.

### Migrationskultur:

Es werden 3 bis 4 Partikel in eine Vertiefung einer Gewebekulturplatte mit 6 Vertiefungen verteilt und mit 3 ml Medium aufgefüllt, bzw. 6 bis 7 Partikel pro 25 cm² Wachstumsfläche mit 7 ml Medium. Die Inkubation erfolgt im Brutschrank bei 37°C und 5% CO₂. Zweimal pro Woche sollte das Medium gewechselt werden, dabei werden die Zellen unter dem Phasenkontrastmikroskop inspiziert. Nach 5 bis 9 Tagen sind erste Zellen zu erkennen, nach 10 bis 14 Tagen ein subkonfluenter Zellrasen mit 65 bis 75% Bodenflächenbedeckung.

### Stromale Zellkultur:

Zunächst wird der Knochen von Muskel- und Bindegewebsresten befreit. Mit Hilfe einer Schere und einer Pinzette wird die Spongiosa in möglichst kleine Stückchen zerkleinert. Die Spongiosa-Fragmente können in ein 50 ml Zentrifugenröhrchen mit Schraubverschluß gegeben und darin gewogen werden. Enthält das Material viel rotes Knochenmark, so kann man später pro 4 bis 6 g Spongiosa eine 75 cm² Gewebekulturflasche beschicken. In das 50 ml Zentrifugenröhrchen mit Schraubverschluß werden nun zur zerkleinerten Spongiosa circa 25 ml Medium 1 (Basalmedium mit Antibiotika, z.B. Medium 199, Fa. Gibco, 100 IU/ml Penicillin, 100 µg/ml Streptomycin) gegeben und durch Vortexen (hochfrequentes Rütteln, circa 30 Sekunden, höchste Stufe) die Zellen herausgelöst. Der Überstand wird in andere Schraubgefäße überführt. Dieser Schritt wird so lange wiederholt, bis das Medium nach Vortexen nicht mehr trüb wird. Zuletzt kann noch eine Trypsin- (und/oder Kollagenase-)Behandlung (circa 10 Minuten, 37°C) durchgeführt werden, um weitere Zellen zu gewinnen. Die erhaltenen Zellsuspensionen werden 10 Minuten bei 250g und 4°C zentrifugiert. Die Überstände werden verworfen, die Zellpellets in Medium resuspendiert und auf Kulturflaschen verteilt. Die gespülten Knochenstückchen können gegebenenfalls in einer separaten Kulturflasche zur Anzüchtung restlicher Zellen verwendet werden (nach Trypsinisierung sind diese aber gut mit Medium zu spülen).

Um die Zellzahl zu bestimmen werden nach der Resuspension der Zellpellets 50 µl der Suspension in ein Eppendorf-Gefäß überführt und mit 46 µl Trypanblau gemischt. Durch Zugabe von 4 µl Essigsäure werden die Erythrocyten lysiert. Die kernhaltigen Zellen werden in einer Neubauer-Zellkammer (Glaskammer zum Zählen von Zellen unter dem Mikroskop) ausgezählt. Anschließend werden etwa 10⁷ Zellen der Zellsuspension pro 75 cm² Kulturflasche verteilt. Der Mediumwechsel erfolgt nach circa 5 Tagen, später zweimal pro Woche. Erste ahärente Zellen sind am zweiten Tag erkennbar, Kolonien ab Tag 4 bis 5. Subkonfluenz wird zwischen Tag 9 und 11 erreicht.

### b) Isolierung und Selektion der Knochenvorläuferzellen aus Knochenmarkaspirat

Nach örtlicher Betäubung wird eine sterile Knochenmarkpunktion durchgeführt. Durch Aspiration mit einer großvolumigen Kanüle und einer Heparin-benetzten Spritze werden etwa 15 bis 20 ml Blut aus dem hinteren Beckenkamm entnommen. Die Probe wird bei etwa 400 g zentrifugiert, der Überstand abgenommen und das Zellpellet in 5 ml serumfreien Medium 1 suspendiert. Anschließend wird eine Dichtegradientenzentrifugation über einen 70%-Percollgradienten (Percoll ist der Handelsname einer viskösen Lösung mit einer erhöhten Dichte gegenüber Wasser) oder einem Ficoll-Kissen (Ficoll ist eine visköse Lösung mit einer höheren Dichte als Wasser, die chemische Zusammensetzung unterscheidet sich von Percoll, wirkt jedoch nach dem gleichen Prinzip. Beide Lösungen werden zur Dichtezentrifugation benutzt.) bei 400 g für 30 Minuten bei 4°C durchgeführt, um die Roten Blutkörperchen von den Mesenchymalen Zellen zu trennen. Schließlich werden die Mesenchymzellen in einer Gewebekulturflasche ausplattiert und vermehrt.

Der osteoblastische Phänotyp der Zellen kann durch Nachweis der knochenspezifischen Proteine Alkalische Phosphatase und Osteocalcin im Kulturmedium oder durch immunhistochemische Färbungen von Kontrollkulturen nachgewiesen werden.

### Bestimmung der alkalischen Phosphatase-Aktivität

Die Zellen werden zweimal mit PBS gewaschen. Pro Ansatz einer Gewebekulturschale mit sechs Vertiefungen werden 2 ml alkalische Phosphatase-Substratpuffer zugegeben (Rotipuran®, Fa. Roth, 50 mM Glycin, 1 mM MgCl₂, 5 mM p-Nitrophenylphosphat, pH 10,5). Es wird 15 Minuten bei 37°C inkubiert. Die Inkubationszeit kann von 5 bis 20 Minuten variiert werden. Anschließend wird die inkubierte Pufferlösung in einer Küvette 1:1 mit 1 M NaOH gemischt. Schließlich wird die Absorption bei 405 nm bestimmt. Die Enzymaktivität wird angegeben als Absorption/Zellzahl.

### Osteocalcinfärbung

Nach Entfernung des Mediums werden die Zellen einmal mit PBS gewaschen. Anschließend werden die Zellen mit der Lösung A des FIX & PERM-Kit (Handelsname eines Kits, mit dem Zellen fixiert und die Membran permeabilisiert werden kann, so daß Farbstoffe und Eiweiße in das Zellinnere eindringen können. Cell Permeabilisation Kit, Fa. AN DER GRUB, erhältlich von Fa. Dianova) für 15 Minuten fixiert, die Lösung wird anschließend abgesaugt. Lösung B des FIX & PERM-Kit wird zusammen mit dem Primärantikörper (rabbit anti human osteocalcin, Fa. PAESL + LOREI; 1:25 in PBS verdünnt) im Verhältnis 1:1 für 2,5 Stunden lichtgeschützt auf den Monolayer pipettiert, danach wird zweibis dreimal mit PBS gespült. Inkubation mit dem Sekundärantikörper (FITC - conjugated goat and rabbit IgG, Fa. Sigma, 1:80 in PBS) erfolgt lichtgeschützt für eine Stunde. Der Primärantikörper erkennt nach dem Schlüsselloch-Prinzip ein bestimmtes Epitop. Der Sekundärantikörper erkennt dann bestimmte Domänen des gebundenen Primärantikörpers. Im Anschluß wird zwei- bis dreimal mit PBS gespült. Anschließend werden die Zellen sofort mit einem Immunfluoreszenzmikroskop mit eingestellter FITC-Anregung untersucht.

### Alkalische Phosphatasefärbung

Zur Färbung wurde ein Kit der Fa. Sigma Diagnostics, Katalog Nr. 86-R verwendet. Zunächst werden folgende Lösungen hergestellt: Die Fixierlösung entsteht durch Mischen von 65 ml Aceton, 25 ml "Citrate Solution" und 8 ml 37% Formaldehyd. Die Lösung ist in einer Glasflasche bei 4°C bis zu vier Wochen haltbar. Die "Diazonium Salt Solution" wird durch Mischen von jeweils 1 ml "FRV Alkaline Solution" und "Sodium Nitrite Solution", 2-minütige Inkubation bei Raumtemperatur und Zugabe des Gemisches zu 45 ml H₂O hergestellt. Schließlich stellt man die "Alkaline Dye Mixture" durch Zugabe von 1 ml "Naphthol AS-BI Alkaline Solution" zu obiger "Diazonium Salt Solution" her (vor Licht schützen). Die Fixierlösung wird auf Raumtemperatur gebracht, dann werden die Zellen 30 Sekunden fixiert. Anschließend werden die Zellen 45 Sekunden mit H₂O gespült, es ist darauf zu achten, daß die Zellen nicht austrocknen. In der Folge inkubiert man die Zellen 15 Minuten mit "Alkaline Dye Mixture" bei Raumtemperatur im Dunkeln. Anschließend wird 2 Minuten mit H₂O gespült. Die Gegenfärbung erfolgt mit "Hämatoxyline Solution". Hämatoxylin ist ein Farbstoff, der Gewebe bei der mikroskopischen Untersuchung rot anfärbt. "Hämatoxyline Solution" wird aufgetropft und 2 Minuten auf den Zellen belassen, dann wird gut mit Wasser gespült. Die Zellen werden an der Luft getrocknet und können unter dem Lichtmikroskop betrachtet werden.

### Beispiel 3: Isolierung von Fibroblasten

Ein Stück Hautgewebe wird von Fettgewebe befreit und in etwa 0,5 cm² große Stücke geschnitten. Die Hautstücke werden in 2,5 U/ml Dispase entweder 3 h bei 37°C oder 16 h bei 4°C inkubiert. Anschließend wird die Epidermis abgezogen und verworfen. Die Stücke werden mit einem Skalpell so gut wie möglich zerkleinert. Der entstehende Brei wird dann mit einer Enzymlösung, enthaltend ca. 90 U/ml Kollagenase und ca. 140 U/ml Hyaluronidase versetzt und 3 h bei 37°C geschüttelt. Anschließend wird der Brei bei 300 g für 30 Minuten bei 4°C zentrifugiert, der Überstand verworfen, der Bodensatz in 5 ml Medium 2 (Medium 1, 10% (v/v) Serum; bei therapeutischem Einsatz wird autologes, humanes Serum verwendet, bei Experimenten fötales Kälberserum, erhältlich von Fa. Gibco) aufgenommen und in eine 25 cm²-Gewebekulturflasche gegeben.

Sollten noch viele unverdaute Stücke vorhanden sein, wird der Bodensatz anstatt in Medium in 5 ml ca. 185 U Kollagenase/ml Pufferlösung aufgenommen, in eine 25 cm²-Kulturflasche gegeben und über Nacht in einem Zellkultur-Brutschrank inkubiert. Am nächsten Tag wird die Kollagenase-haltige Suspension abgenommen, zentrifugiert, der Bodensatz in Medium 2 aufgenommen und in dieselbe Flasche zurückgegeben.

### Beispiel 4: Herstellung der Knorpelkomponente

### a) Kollagenschwämme

Die Zellsuspension wird auf etwa 3 x 10⁷ Zellen/ml eingestellt. Kollagenvliese werden in etwa 1 x 1 cm große Stückchen geschnitten (steril). Die Vliese werden mit einer Insulinspritze beimpft. Dabei reichen 2 ml Zellsuspension für 8 bis 10 Kollagenstückchen aus. Nach drei Stunden im Brutschrank werden die Stückchen auf eine Gewebekulturplatte mit sechs Vertiefungen verteilt und mit jeweils 8 ml Nährmedium aufgefüllt. Das Medium wird alle zwei Tage gewechselt.

### Tierversuch:

Primäre Rinder-Chondrozyten wurden als Suspension in Schwämme aus Rinder-Kollagen Typ 1 geimpft. Die Konstrukte wurden sieben Tage bei 37°C und 5% CO₂ im Brutschrank kultiviert und anschließend Nacktmäusen subkutan implantiert. Kontrollgruppen waren Kollagenschwämme ohne Zellen und Chondrozytensuspensionen ohne Trägermaterial. Die Explantation erfolgte nach drei, sechs und neun Wochen. Die Konstrukte wurden histologisch-immunhistochemisch untersucht. Die biomechanische Prüfung erfolgte durch einen "confined compression test" zur Bestimmung der hydraulischen Permeabilität und des Kompressionsmoduls (Biomechanisches Testverfahren, bei dem auf eine zu prüfende Probe definierte, zyklische Drücke ausgeübt und Kraftrelaxationsmessungen durchgeführt werden). Als Ergebnis zeigte sich morphologisch hyaliner Knorpel mit immunhistochemischem Nachweis von knorpeltypischem Kollagen Typ 2. In den Kontrollen wurde der leere Kollagenschwamm drei Wochen resorbiert. Die Zellsuspension entwickelte nur kleine Knorpelstücke. Die Steifigkeit der Konstrukte nahm im zeitlichen Verlauf zu (1,99 Mpa in Woche 9), war jedoch gegenüber nativem Knorpel (5,25 Mpa) verringert. Die hydraulische Permeabilität zeigte signifikant höhere Werte als die Kontrolle (2,69 x 10⁻¹⁴ gegenüber 3,0 x 10⁻¹⁵ m⁴/Ns).

### b) Fibrinsuspension

Die Knorpelzellen werden gelöst und nach Zentrifugation in der Thrombinkomponente eines Zweikomponentenfibrinklebers in einer Dichte von 2-3 x 10⁷ Zellen/ml eingebracht. Anschließend wird durch synchrones Spritzen mittels einer Doppelspritze die zellhaltige Thrombin- mit der Fibrinogenkomponente vermischt und in eine vorgefertigte Kulturform gegossen. Es entsteht durch Präzipitation des Fibrinogen zu Fibrin ein fester "Clot". Schließlich wird mit HAM's F12-Medium (Hepes-Modifikation, Zugabe von Hepes-Pufferlösung zu einem Nährmedium) überschichtet und im Brutschrank bei 37°C und 5% CO₂ inkubiert.

### Tierversuch:

Primäre Chondrozytenkulturen werden aus Rippenknorpelbiopsien etabliert und mit einer Dichte von 2 x 10⁷ Zellen/ml in der Thrombinkomponente eines Fibrinklebers suspendiert. Anschließend wird durch eine Doppelspritze die Fibrinogenkomponente mit der chondroblastenhaltigen Thrombinkomponente (4 IE Thrombin/ml) zusammengebracht und in eine strukturierte, anatomisch angepaßte Form gegossen. Der Fibrinkleber verfestigt sich in Abhängigkeit von der Thrombinkonzentration innerhalb von 5-10 Minuten in der gewünschten Form.

### Tierversuch:

Die Konstrukte wurden Nacktmäusen subkutan implantiert. Kontrollgruppen wurden "Fibrinclots" ohne Zellen und Chondrozytensuspensionen ohne Fibrin implantiert. Die Explantation erfolgte nach drei, sechs und neun Wochen. Die Konstrukte wurden histologisch-immunhistochemisch untersucht. Die biomechanische Prüfung erfolgt durch einen "confined compression test" zur Bestimmung der hydraulischen Permeabilität und des Kompressionsmoduls.

Als Ergebnis fanden sich in der Gruppe mit Knorpelzellen in Fibrinkleber mikroskopisch neu gebildetes Knorpelgewebe, in den Kontrollgruppen war kein Knorpelgewebe zu finden. Histologisch zeigten die Knorpelkonstrukte die Morphologie von hyalinem Knorpel mit immunhistochemischem Nachweis von Kollagen Typ 2. Die Knorpelkonstrukte zeigten bei der biomechanischen Prüfung im "confined compression test" eine Steifigkeit (Kompressionsmodul) von 0,59 Mpa und eine hydraulische Permeabilität von 1,03 x 10⁻¹⁴ m⁴/Ns.

### Beispiel 5: Herstellung der Knochenkomponente

### a) Herstellung der Zell-Biomaterialkonstrukte

Die Trägermaterialien werden eine Woche vor Besiedelung mit Zellen in Kulturmedium gewässert, um eine Rehydrierung und pH-Stabilität zu erreichen.

Durch Trypsinisierung werden die als Monolayerkultur gezüchteten Zellen aus der Kulturschale abgelöst und nach Zentrifugation und Zählung in einem geringen Volumen als Einzelzellsuspension suspendiert. Das Medium wird abgenommen, ein Teil in ein 1,8 ml-Kryoröhrchen abgefüllt und eingefroren, der Rest wird verworfen. Aus den Überständen wird mittels eines Enzyme-Linked-Immuno-Sorbence-Assay (ELISA) Osteocalcin und alkalische Phosphatase bestimmt. In einer Gewebekulturplatte mit sechs Vertiefungen wird mit 0,5 ml Trypsin/EDTA-Lösung (37°C; 0,025% Trypsin) pro Vertiefung das restliche Medium abgespült. Anschließend werden mit 1 ml Trypsin/EDTA pro Vertiefung die Zellen vom Boden gelöst. Das Trypsin sollte nicht länger als fünf bis acht Minuten auf den Zellen verbleiben, um irreversible Schädigung zu vermeiden. Nachdem sich die Zellen vom Boden gelöst haben, wird mit 1 ml serumhaltigem Kulturmedium 2 neutralisiert (vorgewärmt). Die Zellsuspension wird in einem Zentrifugenröhrchen mit Schraubverschluß gesammelt und bei ca. 300g und 4°C für 10 Minuten zentrifugiert. Der Überstand wird abgesaugt und das Zellpellet mit 1 ml flüssiger oder visköser Lösung suspendiert und ausgezählt.

Die flüssige oder visköse Zellsuspension wird in einer Aspirationskammer in die dreidimensionalen Trägermaterialien durch Unterdruck ohne Volumenverlust eingezogen, so daß die gesamte innere Oberfläche besiedelt wird.

### b) Ex vivo-Züchtung der Konstrukte

Die Zell-Biomaterialkonstrukte werden in eine spezielle sterile Perfusionskammer gebracht und eingespannt, so daß ein unidirektioneller Durchfluß des Kulturmediums gewährleistet ist. Neben der Zufuhr von immer frischem Medium werden die Zellen durch den Flüssigkeitsstrom mechanisch stimuliert. Das Kulturmedium (Osteoblastenmedium, z.B. BGJ-B-Medium, Fa. Gibco, mit 100 IU/ml Penicillin, 100 µg/ml Streptomycin, 10% (v/v) Serum; bei therapeutischem Einsatz wird autologes, humanes Serum verwendet, bei Experimenten fötales Kälberserum) wird durch Zusatz von 10⁻⁸ bis 10⁻¹⁰ M Dexamethason, 50 µg/ml Vitamin C und 40 ng/ml Vitamin D3 im zuführenden Teil angereichert, um die Zellen zur Matrixsynthese zu stimulieren.

### c) Analyse der Konstrukte in der Kulturphase

Zur Messung des Zellmetabolismus wird den Konstrukten ein Tetrazoliumsalz "XTT" zugesetzt. Der Test beruht auf der kolorimetrischen Biotransformation des Salzes zu dem intensiv gelb gefärbten Formazan in den Mitochondrien. Die Formazankonzentration wird zu bestimmten Zeitpunkten nach der Zugabe der Lösung photometrisch bei 450 nm Wellenlänge gemessen. Das Ergebnis korreliert mit dem Zellmetabolismus bzw. der Proliferation der Zellen (Cell Proliferation Kit, Firma Roche Diagnostics, Mannheim).

Die Adhäsion kann durch "Environmental Scanning Electron Microscopy" (ESEM) kontrolliert werden. Die "Environmental Scanning Electron Microscopy" ist ein technisches Verfahren der Elektronenmikroskopie, bei dem Zellen nicht fixiert werden müssen, sondern lebend im Kulturmedium durch Elektronenmikroskopie betrachtet werden können. 48 Stunden nach Besiedelung der Biomaterialien werden die Konstrukte auf einen Träger geklebt und in eine ESEM-Kammer (Freiburger Materialforschungsinstitut) gebracht. Hiermit kann in der Vergrößerung eines Elektronenmikroskopes die Anhaftung der Zellen auf der Materialoberfläche dargestellt werden.

### d) Analyse der Konstrukte am Ende der Kulturphase

Nach zwei bis vier Wochen in der Perfusionskammer werden die Konstrukte fixiert und durch die klassische HE-Färbung histologisch auf eine neue Matrixbildung auf den Trägermaterialien untersucht. Durch die spezielle Richardson-Levaletzko-Färbung wird neues Knochengewebe intensiv blau angefärbt.

Durch Immunhistochemie wird Kollagen 1 als organischer Hauptbestandteil des Knochengewebes nachgewiesen (z.B. durch die Avidin-Biotin-Methode).

Durch Immunfluoreszenz mit FITC-markierten Antikörpern wird Osteocalcin als spezifischer Knochenmarker in dem neuen Gewebe dargestellt.

### e) Zusatz angiogener Wachstumsfaktoren zur Knochenkomponente (optional)

Zur Induktion der Gefäßneubildung können Wachstumsfaktorproteine (VEGF, bFGF, Ang I oder Ang II) der Knochenkomponente zugesetzt werden. Zunächst wird wie in Beispiel 2 beschrieben eine Osteoblastenzellkultur etabliert. Die subkonfluenten Zellen in einer 75 cm² Zellkulturflasche werden mit 1 ml 0,025% Trypsin/EDTA-Lösung 5 Minuten trypsinisiert. Nach Resuspension in 2 ml Medium mit 10% FCS und 5 Minuten Zentrifugation bei 1000 Upm und 4°C werden die Zellen in 100 µl Medium resuspendiert und gezählt. 20000 Osteoblasten der Zellpassage 1 bis 3 werden in 200 µl Fibrinogenlösung suspendiert (66 mg Fibrinogen in 1 ml αMEM oder Medium 199 oder BGJ-B-Medium ohne Serum mit 100 U/ml Penicillin und 100 mg/ml Streptomycin gelöst; 0,1 bis 10% ε-Amino-n-capronsäure). Anschließend werden 40 µl einer 40 mM Calciumchloridlösung enthaltend 1,25 I.E. Thrombin/ml und 0,1 bis 10 ng/ml rekombinanten Wachstumsfaktor zur Osteoblasten-Fibrinogensuspension gegeben. Das Gemisch wird anschließend in eine Zellkulturschale gespritzt (beispielsweise 48 Well Platte). Nach Zugabe von 760 ml Kulturmedium BGJ-B mit 10% FCS und 100 U/ml Penicillin und 100 mg/ml Streptomycin wird das Zellkonstrukt im Wärmeschrank bei 37°C, 5% CO₂ und 100% Luftfeuchte kultiviert.

### f) Herstellung einer Osteoblasten-Endothelzell-Fibrinogensuspension OEFS (optional)

Eine Osteoblasten-Zellkultur wird wie in Beispiel 2 beschrieben etabliert. Die subkonfluente Osteoblastenkultur in einer 75 cm² Zellkulturflasche wird mit 1 ml 0,025% Trypsin/EDTA-Lösung 5 Minuten trypsinisiert. Nach Resuspension in 2 ml Endothelzellmedium ohne Serum und 5 Minuten Zentrifugation bei 1000 Upm und 4°C werden die Zellen in 100 µl Endothelzellmedium resuspendiert und gezählt. 1 bis 10 x 10⁴ (vorzugsweise 20000) Osteoblasten der Zellpassage 1 bis 3 werden in 200 µl Fibrinogenlösung suspendiert (66 mg Fibrinogen in 1 ml Endothelzellmedium mit 100 U/ml Penicillin, 100 mg/ml Streptomycin und 0,1 bis 10% ε-Amino-n-capronsäure). Parallel dazu wird eine Endothelzellkultur nach Standardprotokoll als mikrovaskuläre Endothelzellkultur etabliert. Die subkonfluente Endothelzellkultur in einer 75 cm² Kulturflasche wird mit 1 ml 0,025% Trypsin/EDTA-Lösung 5 Minuten trypsinisiert. Nach Resuspension der Zellen 2 ml Endothelzellmedium ohne Serum und 5 Minuten Zentrifugation bei 1000 Upm und 4°C werden die Zellen in 100 µl Endothelzellmedium resuspendiert und gezählt. 1 bis 10 x 10⁴ (vorzugsweise 20000) Endothelzellen der Zellpassage 1 bis 3 werden in 200 µl der oben genannten Fibrinogenlösung suspendiert. Schließlich werden die Osteoblasten-Fibrinogensuspension und die Endothelzell-Fibrinogensuspension miteinander durch Resuspension vermischt. Die Osteoblasten-Endothelzell-Fibrinogensuspension (OEFS) wird in ein poröses, dreidimensionales Trägermaterial, z.B. autoklavierte Spongiosa, eingesaugt. Pro ml OEFS werden 40 µl Calciumchlorid-Thrombinlösung zugesetzt (1,25 I.E. Thrombin pro ml, 40 mM Calciumchlorid). Das Konstrukt wird in eine Kulturschale oder eine Perfusionskammer eingebracht. Anschließend wird Endothelzellmedium mit 10% FCS, 100 U/ml Penicillin und 100 mg/ml Streptomycin zugegeben. Es erfolgt Kultivierung im Wärmeschrank bei 37°C, 5% CO₂ und 100% Luftfeuchte für 1 bis 7 Tage bis zur Fusion mit der Knorpelkomponente.

Durch Zugabe der Calciumchlorid-Thrombinlösung bildet sich ein dreidimensionales Fibrinnetzwerk mit haftenden Osteoblasten und Endothelzellen in der Fibrinmatrix, welche das poröse Trägermaterial ausfüllt. Im Lichtmikroskop (100-fache Vergrößerung) zeigt sich die Ausbildung des osteoblastischen Phänotyps mit dendritischen Zellausläufern und der Aufbau von interzellulären Verbindungen der Osteoblasten. Dazwischen liegen kleinere Endothelzellen, die in vivo proliferieren und sich zu Kapillaren organisieren können.

Die Vitalität kann durch Trypanblaufärbung nach 7 Tagen untersucht werden. Dazu wird der Überstand des Kulturmediums abgesaugt, es werden 50 µl Trypanblaulösung zugegeben, anschließend wird das Präparat unter dem Lichtmikroskop kontrolliert. Nach 7 Tagen finden sich nur wenig abgestorbene Zellen.

### Beispiel 6: Herstellung von Bandkomponenten

Fibroblasten werden als Suspension mit 1 x 10⁷ bis 5 x 10⁷ Zellen/ml Medium auf bandförmige Materialien geimpft und in Dulbecco's Minimal Essential Medium (DMEM)-Medium mit 10% autologem Serum bei 37°C und 5% CO₂ in Kultur gehalten.

### Beispiel 7: Fusion der Einzelkomponenten

Primäre Chondrozytenkulturen wurden aus Rippenknorpelbiopsien etabliert und mit einer Dichte von 2 x 10⁷ Zellen/ml in der Thrombinkomponente eines Fibrinklebers suspendiert. Anschließend wird durch eine Doppelspritze die Fibrinogenkomponente mit der chondroblastenhaltigen Thrombinkomponente zusammengebracht und in eine strukturierte, anatomisch angepaßte Form gegossen. Der Fibrinkleber verfestigt sich in Abhängigkeit von der Thrombinkonzentration innerhalb von 5-10 Minuten in der gewünschten Form. Parallel werden primäre Osteoblastenkulturen aus Beckenkamm-Biopsien (siehe Beispiel 2) hergestellt und der osteoblastische Phänotyp durch Alkalische Phosphatase und Osteocalcin nachgewiesen. Die Zellen werden auf dreidimensionalen, porösen Biomaterialien (z.B. bovine oder humane Spongiosa) als Suspension mit 1 x 10⁶ bis 1 x 10⁷ Zellen/ml viskösem Gel oder flüssigem Medium durch Vakuumaspiration aufgebracht. Die Adhäsion der Zellen wird durch Elektronenmikroskopie und die Proliferation durch XTT-Stoffwechseltests kontrolliert. Die einzelnen Konstrukte werden getrennt für drei Tage in vitro bei 37°C und 5% CO₂ kultiviert. Im Anschluß erfolgt die Fusion der Knochen- und Knorpelkomponente durch Fibrinklebung.

### Die Fusion kann auch abgewandelt wie folgt erreicht werden:

Während des Prozesses der Herstellung der Knorpelkomponente und der Abbindung des Fibrinklebers wird das leere, vorgeformte und angepaßte Knochenträgermaterial leicht in die Knorpelschicht eingedrückt, so daß es fest eingebunden wird.

Im zweiten Schritt erfolgt dann die Besiedelung der Knochenkomponente durch die Osteoblastensuspension in einem viskösem Gel oder flüssigem Medium.

Als Ergebnis eines derartigen Versuchs waren Knorpel- und Knochenkomponente fest miteinander fusioniert. Die Knorpelfläche war glatt und war deutlich von der Knochenkomponente abgrenzbar. Makroskopisch bestand eine korrekte, stabile anatomische Gelenkform entsprechend den natürlichen anatomischen Gegebenheiten.

### Knorpel-Knochenkonstrukte mit Bandapparat:

Die oben beschriebenen Knorpel-Knochenkonstrukte werden mit Bandkonstrukten verbunden, indem ein 0,5 mm großer Bohrkanal durch die Knochenkomponente gebohrt wird und das Bandkonstrukt, bestehend aus Fibroblasten auf einem Kollagenband, hindurchgezogen wird.

Das Bandkonstrukt ist fest integriert und kann zwei Knorpel-Knochenkonstrukte so verbinden und halten, daß die Knorpelflächen artikulieren und sich gegeneinander bewegen können.

### Tierversuche:

Ca. sechs bis acht Wochen alte Nacktmäuse wurden in einer Narkosekammer mit einem Isofluran®-Sauerstoffgemisch (3% Isofluran® in 100% O₂, Fluß 4 l/min.) betäubt. Unter Aufrechterhaltung der Narkose mit einer Inhalationsmaske (1,5 - 2% (v/v) Isofluran® in 100 % O₂, Fluß 0,5 bis 1 l/min.) wurden die Tiere mit Betaisodona® abgewaschen und das Operationsgebiet steril abgedeckt. Es erfolgte ein ca. 2 cm langer, längs verlaufender Hautschnitt im Bereich des Rückens, Spreizen mit der Präparationsschere und Schaffen einer Hauttasche. Das osteochondrale Transplantat (ca. 2 x 0,5 x 0,5 cm) wurde eingelegt und die Wunde mit Einzelknopfnähten verschlossen. Ein steriler Wundverband wurde angelegt. Der Eingriff dauerte etwa 15 Minuten. Die Wunden der Tiere wurden bis zur gesicherten Wundheilung täglich kontrolliert.

Die Nacktmäuse erhielten schließlich eine letale Dosis CO₂ per inhalationem am 21., 42. bzw. 63. Tag postoperativ. Die osteochondralen Konstrukte wurden mit dem umgebenden Gewebe herauspräpariert und danach histologisch und immunhistochemisch aufgearbeitet. Als Ergebnis waren in den fusionierten osteochondralen Konstrukten die Knorpel- und Knochenkomponente fest miteinander verbunden. Histologisch zeigte die Knorpelschicht die Morphologie von hyalinem Knorpelgewebe mit immunhistochemischem Nachweis von Kollagen Typ 2. In der Knochenkomponente fand sich nach acht Wochen ein appositionelles Knochenwachstum mit eingesproßten Kapillaren. Die Knorpelkomponente zeigte bei der biomechanischen Prüfung im "confined compression test" eine Steifigkeit (Kompressionsmodul) von 0,59 Mpa und eine hydraulische Permeabilität von 1,03 x 10⁻¹⁴ m⁴/Ns.

### Beispiel 8: Lipofektion von Osteoblasten mit EGF-Plasmid

Humane Osteoblasten werden am Tag vor der Transfektion auf Gewebekulturbehälter verteilt. Dabei wurden Gewebekulturschalen mit 6 Vertiefungen (Fa. Costar, Kat. Nr. 3506, 9,6 cm² pro Vertiefung), im folgenden "6-well-Platten" genannt, und Zellkultureinsätze (Falcon Cell Culture Insert, Fa. Becton Dickinson, PET-Membran mit 1,6 x 10⁶ Poren (1 µm) pro cm², Kat. Nr. 3102, 4,2 cm² Fläche) verwendet.

Es folgen zwei Protokolle, mit denen hohe Expressionen erreicht werden.

### a) Transfektion subkonfluenter Monolayerkulturen in 6-well-Platten

Zur Herstellung der Transfektionslösung werden pro Ansatz 230 µl Medium 1 ohne Zusätze, 15 µl Escort®-Reagens (Fa. Sigma) und 6 µg Plasmid-DNA (enthaltend das Gen für humanes EGF) gemischt. Nach 15 min Inkubation bei Raumtemperatur wird der Lösung pro Ansatz 2 ml Medium 2 hinzugefügt. Das Kulturmedium wird von den Zellen entfernt und das Transfektionsgemisch wird zu den Zellen gegeben (2,25 ml/Vertiefung). Danach werden die Zellen 12 h bei 37°C und 5% CO₂ im Brutschrank inkubiert. Das Transfektionsgemisch wird von den Zellen entfernt, die dann mit Medium 2 gewaschen werden. Schließlich wird zur Weiterkultivierung ca. 3 ml Vollmedium in jede Vertiefung gegeben. Das Medium wird täglich gewechselt, von den Überständen werden Proben bei -20°C aufbewahrt, die dann zur Bestimmung des EGF-Gehalts verwendet werden. Die Epidermal growth factor (EGF)-Konzentration wurde durch einen ELISA bestimmt (Quantikine™, R&D Systems, Kat. Nr. DEG-00). Hierzu müssen die Proben gegebenenfalls vorher 1:10-1:15 in Medium verdünnt werden, da der Meßbereich des ELISA 0-250 pg/ml beträgt.

Die Analyse ergab, daß EGF über 10 Tage mit einem Maximum am 5. Tag sezerniert wurde.

### b) Transfektion subkonfluenter Monolayerkulturen in Zellkultureinsätzen ("Trennkammerversuch")

Bei den Zellkultureinsätzen handelt es sich um Einsätze, die eine poröse Membran aufweisen, an die die Zellen adhärieren können. Der Einsatz wird in eine Gewebekulturplatte mit (meist 6) Vertiefungen eingesetzt, so daß die "apikalen" Zellen in dem Einsatz ca. 3 mm von den "basalen" Zellen in der Vertiefung der Kulturschale räumlich getrennt sind, aber per diffusionem Signalstoffe über die permeable Membran austauschen können. Medium wird zur "basalen" und "apikalen" Seite der Zellen gegeben, also in die Vertiefung der Zellkulturschale, in die der Einsatz eingesetzt ist und in den Einsatz selbst. Diese Anordnung kann benutzt werden, um einen parakrinen Effekt sezernierter Wachstumsfaktoren auf andere Zellen zu bestimmen. Dazu werden in den Zellkultureinsatz Osteoblasten ausgesät, die transfiziert werden. Nach der Transfektion werden die Einsätze in Platten transferiert, die mit nicht-transfizierten Zellen besät sind. Da das Medium und Faktoren, die von den transfizierten Zellen ausgeschieden werden, durch den Filter passieren können, kann durch Zellzahl-Bestimmung der nicht-transfizierten Zellen im anderen Kompartiment eine verstärkte Proliferation nachgewiesen werden.

Die Transfektionslösung wird wie in a) hergestellt. Das Kulturmedium wird aus der Vertiefung der Kulturplatte und aus dem Einsatz möglichst vollständig entfernt. In jeden Einsatz werden 2,25 ml Transfektionslösung gegeben. Nach 30-60 min Inkubation im Brutschrank wird 1 ml Vollmedium zugegeben. Falls während der Inkubationsphase die Transfektionslösung rasch durch den Filter des Einsatzes läuft, muß gegebenenfalls früher Medium zugegeben werden. Anschließend wird 12 h bei 37°C und 5% CO₂ inkubiert. Die Transfektionslösung wird abgesaugt, der Einsatz wird ausgiebig mit Medium gewaschen. Der Einsatz kann nun in eine Kulturschale mit Zellen überführt werden. Das Mediumvolumen beträgt insgesamt 4 ml/Vertiefung und Einsatz.

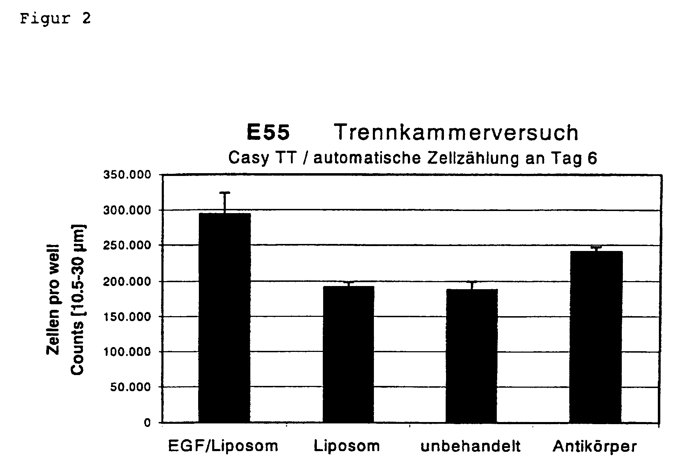

In einem Versuch wurden 10 mit Osteoblasten besäte Einsätze mit EGF-Plasmid transfiziert. Als Kontrollen wurden 10 Inserts mit Zellen nur mit Liposomenlösung ohne Plasmid behandelt, weitere 6 Einsätze wurden völlig unbehandelt gelassen, weitere 6 Einsätze wurden mit EGF-Plasmid transfiziert, nach der Transfektion wurde aber täglich 11,9 µg/ml Medium neutralisierender anti-EGF-Antikörper zugegeben (Fa. R&D, No. AB-236-NA, 1 mg/ml in PBS, pH 7,4, steril). Am Tag 6 nach der Transfektion wurde die Zellzahl der nicht-transfizierten Zellen im unteren Kompartiment durch Zellzählung mit einem "Casy TT"-Zellzähler bestimmt. Mit dem von der Firma Casy hergestellten Gerät können Zellen in Lösungen spektrometrisch gezählt werden. Das Ergebnis ist in Figur 2 gezeigt. Beide Kontrollansätze ("Liposom" und "unbehandelt") zeigen signifikant niedrigere Zellzahlen als der Ansatz, in dem EGF-Plasmid transfiziert wurde("EGF/Liposom"). Das zeigt, daß die transfizierten Zellen einen proliferativen Effekt haben. Die dritte Kontrolle ("Antikörper") zeigt wiederum niedrigere Zellzahlen als der Ansatz mit transfizierten Zellen, aber ohne Antikörper ("EGF/Liposom"). Das beweist, daß der proliferative Effekt auf EGF zurückzuführen ist. Dieses Experiment zeigt, daß durch transiente Lipofektion von Osteoblasten Zellen erzeugt werden können, die Wachstumsfaktoren sezernieren, die das Wachstum anderer Zellen stimulieren können.

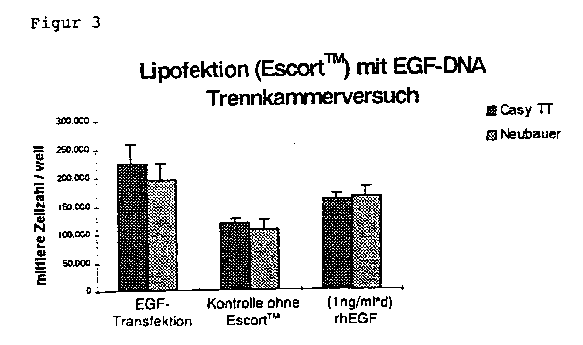

In einem weiteren "Trennkammerversuch" wurde der proliferative Effekt EGF-transfizierter Osteoblasten mit dem proliferativen Effekt von rekombinantem, humanem EGF verglichen, das den Zellen täglich zugesetzt wurde. Am Tag 4 nach Transfektion wurde die Zellzahl der verschiedenen Ansätze bestimmt. Das geschah durch automatische Zellzählung mittels "Casy TT" und durch Auszählen in einer Neubauer-Zählkammer. Das Ergebnis ist in Figur 3 gezeigt. Nach Transfektion mit EGF-DNA ("EGF-Transfektion") ist die Zellzahl des Transfektionsansatzes fast doppelt so hoch wie die des nicht transfizierten Kontrollansatzes ("Kontrolle"). Zugegebenes rekombinantes EGF (1 ng/ml jeden Tag) bewirkt zwar auch verstärktes Wachstum ("rhEGF-Zugabe"), jedoch nicht so stark wie es durch die Transfektion erreicht wird.

### Beispiel 9: Lipofektion von Osteoblasten mit hbFGF-Plasmid

Osteoblasten werden mit einem Plasmid transfiziert, das eine Nucleinsäure enthält, die für humanen "basic fibroblast growth factor" (hbFGF) codiert.

Am Vortag der Transfektion werden die Zellen auf betreffende Kulturbehälter verteilt (z.B. 6-well-Platten oder Zellkultureinsätze für den Trennkammerversuch). Eingesetzt werden je 40000 Zellen pro Vertiefung der 6-well-Platte (9,6 cm² Wachstumsfläche pro Vertiefung) oder pro Zellkultureinsatz (4,2 cm² Wachstumsfläche pro Einsatz). Es folgen zwei Protokolle, mit denen eine hohe Expression der transfizierten Gene erzielt wurde.

### a) Transfektion subkonfluenter Monolayerkulturen in 6-well-Platten (Escort™-Reagens, Fa. Sigma)

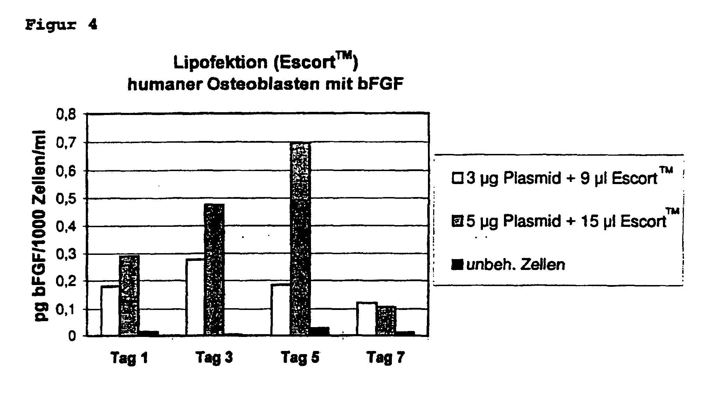

Die Transfektionslösung wird durch Mischen von 115 µl Medium 1, 9 µl Escort™-Reagens (Fa. Sigma) und 3 µg hbFGF-Plasmid pro Ansatz hergestellt. Alternativ können auch 115 µl Medium 1, 15 µl Escort™-Reagens und 5 µg Plasmid gemischt werden. Nach 15 min Inkubation bei Raumtemperatur wird das Kulturmedium von den Zellen entfernt, statt dessen wird 1 ml Medium mit Zusätzen zu den Zellen gegeben, gefolgt von der Transfektionslösung. Anschließend wird 1-2 h bei 37°C und 5% CO₂ inkubiert. Es folgt Zugabe von 2 ml Medium/Vertiefung und 24 h Inkubation bei 7°C und 5% CO₂. Weiterhin wird das Medium täglich gewechselt, wobei Proben des Kulturüberstandes bei - 20°C bis zur Analyse aufbewahrt werden. Die bFGF-Konzentration in den Proben wird durch ELISA bestimmt. Das Ergebnis ist in Figur 4 gezeigt. Bei Verwendung von 3 µg DNA und 9 µl Escort™ ist die FGF-Sekretion am Tag 3 nach der Transfektion am höchsten, bei Verwendung von 5 µg DNA und 15 µl Escort™ am Tag 5 nach der Transfektion. Die ins Medium abgegebene FGF-Menge ist bei Transfektion mit 5 µg DNA höher.

### b) Transfektion subkonfluenter Monolayerkulturen in 6-well-Platten (Fugene®-Reagens)

Die Transfektionslösung wird durch Mischen von Medium ohne Zusätze, Fugene®-Reagens (Fa. Roche Diagnostics) und DNA hergestellt. Es wurden 3 Varianten getestet:

| **Ansatz:** | **A** | **B** | **C** |
|---|---|---|---|
| µl Medium ohne Zusätze: | 97 | 94 | 91 |
| µl Fugene®-Reagens: | 3 | 6 | 9 |
| µg hbFGF-Plasmid: | 3 | 3 | 3 |

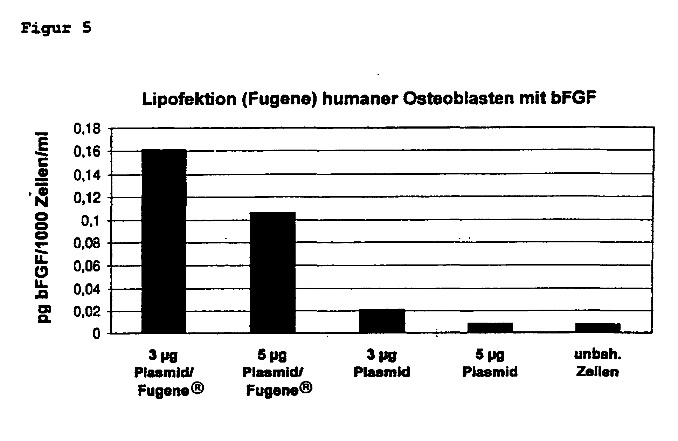

Dabei werden jeweils das Medium und das Fugene®-Reagens zuerst gemischt und 5 min bei Raumtemperatur inkubiert. Anschließend wird die DNA zugegeben, nach Mischen wird weitere 15 min bei Raumtemperatur inkubiert. Das Medium wird von den Zellen entfernt, dann werden pro Vertiefung 3 ml Medium und danach die Transfektionslösung zugegeben. Es folgt eine 24-stündige Inkubation bei 37°C und 5% CO₂ im Brutschrank. Im Folgenden wird täglich das Medium gewechselt, wobei wiederum Proben des Kulturüberstandes bis zur Analyse bei -20°C gelagert werden. Die FGF-Konzentration im Medium wird durch ELISA bestimmt. Auch bei Verwendung des Fugene-Reagens' kann die DNA-Menge variiert werden. Es wurden 3 und 5 µg Plasmid getestet. Das Ergebnis ist in Figur 5 gezeigt. Am Tag 7 nach der Transfektion wird bei Einsatz von 3 µg DNA mehr FGF sezerniert als bei Einsatz von 5 µg DNA ("3 µg Plasmid/Fugene®" und "5 µg Plasmid/Fugene®"). Als Kontrolle wurde auch nur DNA ohne Fugene®-Reagens "transfiziert". Die gemessene FGF-Menge ist deutlich niedriger als bei Verwendung von Fugene® ("3 µg Plasmid" und "5 µg Plasmid"). Als weitere Kontrolle wurde die Fibroblast-Growth-Factor (FGF)-Konzentration im Medium von völlig unbehandelten Zellen ermittelt ("unbeh. Zellen").

Das Ergebnis zeigt, daß Osteoblasten erfolgreich mit hbFGF-DNA transfiziert werden können und daß daraufhin mehr FGF ans Medium abgegeben wird.

### c) Transfektion subkonfluenter Monolayerkulturen in Zellkultureinsätzen ("Trennkammerversuch")

Die Transfektionslösung wird durch Mischen von 115 µl Medium ohne Zusätze, 15 µl Escort®-Reagens und 5 µg hbFGF-Plasmid hergestellt. Nach 15 min bei Raumtemperatur wird 2 ml Medium 2 zugegeben. Das Kulturmedium wird aus dem Zellkultureinsatz, in dem die zu transfizierenden Zellen sind, und aus der Vertiefung der 6-well-Platte, in der sich der Einsatz befindet, möglichst vollständig entfernt. Pro Einsatz werden 2,25 ml Transfektionslösung zu den Zellen gegeben. Es wird 30-60 min bei 37°C und 5% CO₂ im Brutschrank inkubiert. Anschließend wird 1 ml Vollmedium pro Einsatz zugegeben (gegebenenfalls muß früher Medium zugegeben werden, falls die Transfektionslösung schnell durch den Filter des Einsatzes läuft). Es wird nochmals 24 h bei 37°C und 5% CO₂ im Brutschrank inkubiert. Danach wird die Transfektionslösung abgesaugt, der Einsatz wird ausgiebig mit Medium gewaschen. Der Einsatz wird nun in eine Kulturschale mit nicht-transfizierten Zellen eingesetzt, deren Proliferation überwacht wird. Das Medium-Volumen von Einsatz und Kulturschale zusammen beträgt 4 ml.

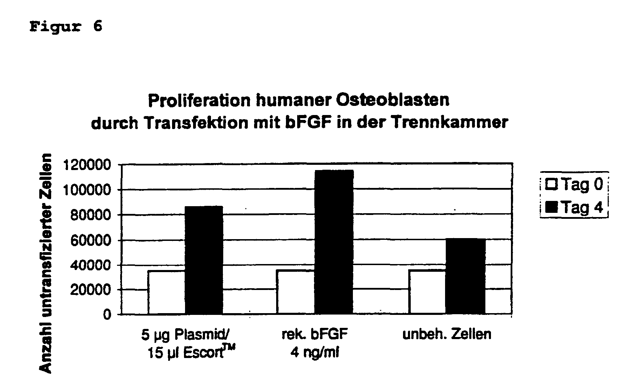

An Tag 4 nach der Transfektion wurde in einem solchen Versuch die Zellzahl der nicht-transfizierten Zellen in den unteren Kompartimenten bestimmt. Figur 6 zeigt das Ergebnis. Die Kontrolle zeigt die natürliche Vermehrung von Zellen in 4 Tagen ("unbeh. Zellen"). Sowohl die Anwesenheit hbFGFtransfizierter Zellen ("5 µg Plasmid/15 µl Escort™") als auch Zugabe von rekombinantem humanem bFGF ("rek. bFGF 4 ng/ml") führen im Vergleich mit der Kontrolle zu erhöhter Proliferation der Zellen. Das zeigt, daß die transfizierten Zellen durch FGF-Sekretion parakrin das Wachstum nichttransfizierter Zellen stimulieren können.

### Beispiel 10: Lipofektion von Osteoblasten mit hVEGF-Plasmid

Subkonfluente Osteoblasten werden mit DNA transfiziert, die für humanen "vascular endothelial growth factor" (hVEGF) codiert. Am Tag vor der Transfektion werden die Zellen auf betreffende Kulturbehälter verteilt. Bei Verwendung von 6-well-Platten werden 50000 Zellen pro Vertiefung eingesetzt.

Die Transfektionslösung wird durch Mischen von Medium ohne Zusätze, Fugene®-Reagens (Fa. Roche Diagnostics) und DNA hergestellt. Es wurden 4 Varianten getestet:

| **Ansatz:** | **A** | **B** | **C** | **D** |
|---|---|---|---|---|
| µl Medium ohne Zusätze: | 94 | 91 | 90 | 85 |
| µl Fugene-Reagens: | 6 | 9 | 10 | 15 |
| µg hVEGF-Plasmid: | 3 | 3 | 5 | 5 |

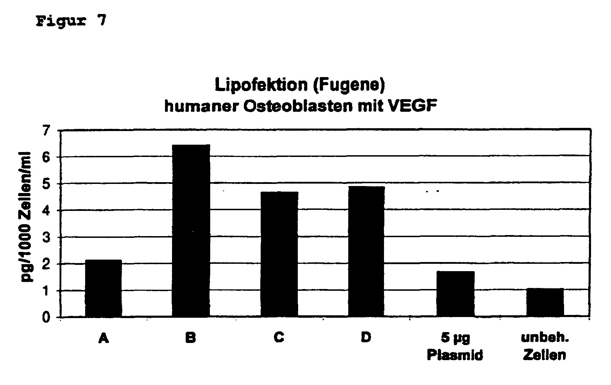

Dabei werden jeweils das Medium und das Fugene-Reagens zuerst gemischt und 5 min bei Raumtemperatur inkubiert. Anschließend wird die DNA zugegeben, nach Mischen wird weitere 15 min bei Raumtemperatur inkubiert. Das Medium wird von den Zellen entfernt, dann werden pro Vertiefung 3 ml Medium und danach die Transfektionslösung zugegeben. Es folgt eine 24-stündige Inkubation bei 37°C und 5% CO₂ im Brutschrank. Im Folgenden wird täglich das Medium gewechselt, wobei wiederum Proben des Kulturüberstandes bis zur Analyse bei -20°C gelagert werden. Die VEGF-Konzentration im Medium wird durch ELISA bestimmt. Das Ergebnis der oben genannten Ansätze ist in Figur 7 gezeigt. Am Tag 7 nach der Transfektion wurde die VEGF-Konzentration bestimmt. Die 4 Ansätze ("A" bis "D") zeigen gegenüber der Kontrolle, die die VEGF-Konzentration im Medium von unbehandelten Zellen zeigt ("unbeh. Zellen"), erhöhte VEGF-Werte. Die Werte sind auch höher als bei Ansätzen, in denen die Zellen nur mit Plasmid, aber ohne Fugene-Reagens behandelt wurden ("5 µg Plasmid").

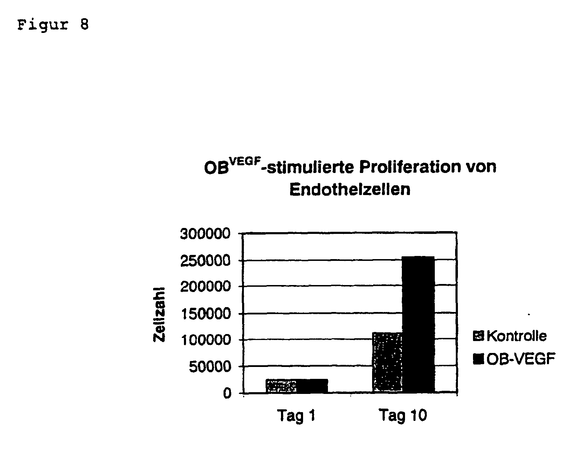

Der induktive Effekt transfizierter Osteoblasten (Ansatz B der Tabelle in Beispiel 10) auf das Wachstum von mikrovaskulären Endothelzellen wurde in der Trennkammer untersucht (Verfahren siehe Beispiel 8b)). Das Ergebnis ist in Figur 8 dargestellt. Es zeigte sich ein deutlich beschleunigtes Wachstum der Endothelzellen gegenüber der Kontrolle ohne transfizierte Osteoblasten.

### Beispiel 11: Elektroporation von Osteoblasten mit EGF-Plasmid

Osteoblasten in Kultur werden durch Trypsinierung abgelöst, zentrifugiert und gezählt. Die Zellsuspension wird auf 1,5·10⁶ Zellen/ml eingestellt. Je 300 µl dieser Suspension werden in eine Elektroporationsküvette (Gene Pulser®/E.coli Pulser™ Cuvette, Cat. No. 165-2086, 0,2 cm gap electrode, Fa. BioRad, CA 94547) überführt. Die gap electrode ist eine Elektrode, mit der bei der Elektroporation elektrische Felder im Nähermedium erzeugt werden. 30 µg DNA (hEGF-Plasmid) werden zu den Zellen in die Küvette gegeben. Eine parallel vorbereitete Kontrollküvette mit Zellen erhält keine DNA. Anschließend werden die Küvetten 10 min auf Eis inkubiert.

Die eigentliche Elektroporation wird mit einem Gerät zur Durchführung von Elektroporationen "Gene Pulser II System" der Fa. BioRad durchgeführt (Cat. No. der main unit: 165-2105). Die Einstellungen sind 150 oder 250 V und 960 µF; die Elektroporationszeit wird von dem Gerät automatisch bestimmt, sie beträgt etwa 4 Sekunden. Die Küvette wird in die Halterung der "shocking chamber" (Kammer, in der Zellen in Näherlösung eingebracht werden und in der elektrische Felder erzeugt werden können) eingespannt, wo die Elektroporation stattfindet. Nach der Elektroporation wird die Küvette aus der Halterung entnommen und 10 min bei 4°C inkubiert. Die Zellen werden in Medium (Osteoblastenmedium, z.B. BGJ-B-Medium, Fa.

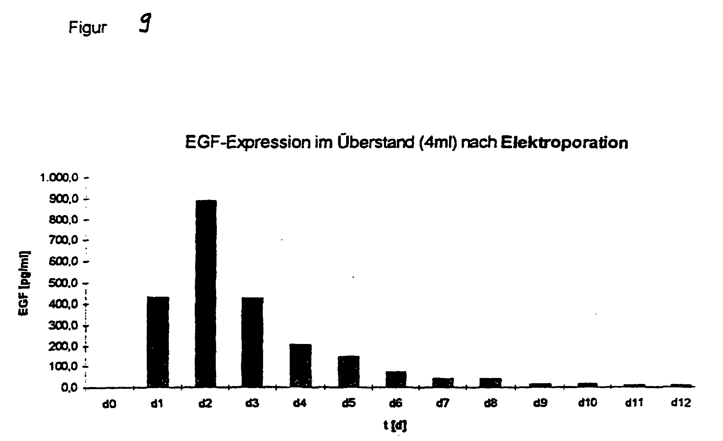

Gibco, mit 100 IU/ml Penicillin, 100 µg/ml Streptomycin, 10% (v/v) Serum; bei therapeutischem Einsatz wird autologes, humanes Serum verwendet, bei Experimenten fötales Kälberserum) resuspendiert und auf Gewebekulturschalen verteilt. Das Medium wird täglich gewechselt (Nachweis von EGF "nicht-kumulativ"). Alternativ kann das Medium nicht gewechselt werden (Nachweis von EGF "kumulativ"). In einem "nicht-kumulativen" Experiment wurden von den 4 ml Kulturüberstand täglich Proben entnommen, die bei -20°C aufbewahrt wurden, bis durch ELISA die EGF-Konzentration bestimmt wurde. Es wurden aber 1,25^{·}10⁶ humane Osteoblasten elektroporiert (250 V, 960 µF; 3,9 s, 20 µg DNA). Das Ergebnis ist in Figur 9 dargestellt. Die gemessene EGF-Konzentration ist in Abhängigkeit von der Zeit in Tagen aufgetragen. Die EGF-Sekretion ins Medium hat am Tag 2 nach Transfektion ein Maximum. EGF-Expression ist mehr als eine Woche lang nachweisbar.

## Patentansprüche

1. Biologisches, zumindest teilweise in vitro hergestelltes Gelenkkonstrukt, das wenigstens folgende Bestandteile umfaßt:
a) wenigstens ein bioverträgliches Trägermaterial;
b) Knorpelgewebe enthaltend Chondrocyten und/oder Chondroblasten und Knorpelsubstanz;
c) Knochengewebe enthaltend Osteoblasten und/oder Osteocyten und Knochensubstanz;
wobei Knorpel- und Knochengewebe fest miteinander verbunden sind, **dadurch gekennzeichnet, daß** das biologische Gelenkkonstrukt eine Gelenkseite aufweist, die Kontakt zu einem anderen Gelenkteil haben kann und deren Oberfläche aus Knorpelgewebe besteht, und eine Ankerseite, die zur Verankerung des Gelenkkonstrukts im Knochenschaft dienen kann und die aus Knochengewebe besteht.

2. Biologisches Gelenkkonstrukt nach Anspruch 1, **dadurch gekennzeichnet, daß** das Knochengewebe zur Verbesserung der Angiogenese ein Wachstumsfaktorprotein, Endothelzellen oder deren Vorläuferzellen, oder mit einem Wachstumsfaktorgen transfizierte Zellen enthält.

3. Biologisches Gelenkkonstrukt nach Anspruch 1, **dadurch gekennzeichnet, daß** die Ankerseite wenigstens einen zylinderförmigen Zapfen aufweist, der mit einem Knochenschaft verbunden werden kann.

4. Biologisches Gelenkkonstrukt nach einem der Ansprüche 1-3, **dadurch gekennzeichnet, daß** es zusätzlich wenigstens eine Bandkomponente umfaßt, die zwei Gelenkteile funktionell miteinander verbinden kann.

5. Biologisches Gelenkkonstrukt nach Anspruch 1 oder 2, **dadurch gekennzeichnet, daß** das Gelenkkonstrukt zum Teilersatz einer Gelenkfläche geeignet ist und als einzelnes präformiertes Konstrukt ausgebildet ist.

6. Biologisches Gelenkkonstrukt nach Anspruch 1 oder 2, **dadurch gekennzeichnet, daß** das Gelenkkonstrukt zum Teilersatz einer Gelenkfläche geeignet ist und als osteochondraler Zylinder ausgebildet ist.

7. Biologischer Gelenkersatz, **dadurch gekennzeichnet, daß** wenigstens zwei Gelenkkonstrukte nach einem der Ansprüche 3-4 mit ihren Gelenkseiten Kontakt zueinander haben und mit ihren Ankerseiten in 2 verschiedenen Knochenschäften verankert werden können.

8. Biologischer Gelenkersatz nach Anspruch 7, **dadurch gekennzeichnet, daß** wenigstens zwei Gelenkkonstrukte durch wenigstens 2 Bandkomponenten verbunden sind.

9. Biologischer Gelenkersatz nach Anspruch 7 oder 8, **dadurch gekennzeichnet, daß** er eine Gelenkkapsel aufweist.

10. Verfahren zur Herstellung eines biologischen Gelenkkonstruktes nach einem der Ansprüche 1-9, das folgende Schritte umfaßt:
a) Bereitstellung einer Knochenkomponente durch Besiedelung eines bioverträglichen Trägermaterials mit Osteöblasten;
b) Bereitstellung einer Knorpelkomponente durch Herstellung einer Suspension von Chondrocyten in einem Medium oder Gel oder durch Besiedelung einer bioverträglichen Trägersubstanz mit Chondrocyten;
c) Verbindung der Knochen- und der Knorpelkomponente, so daß das Trägermaterial in den Knorpel integriert wird;
d) Züchtung: des Konstruktes in vitro, wobei durch Stimulation der Zellen zur Anheftung und zur Synthese ihrer gewebespezifischen extrazellulären Matrix eine biologische Vernetzung der zusammengesetzten Komponenten erreicht wird;

11. Verfahren nach Anspruch 10, **dadurch gekennzeichnet, daß** das Trägermaterial der Knochenkomponente (a) so geformt wird, daß es eine Gelenkseite zur Aufnahme einer Knorpelfläche und eine Ankerseite zur Verbindung mit einem Knochen aufweist.

12. Verfahren nach Anspruch 10 oder 11, **dadurch gekennzeichnet, daß** zur Bereitstellung der Knorpelkomponente (b)
aa) Chondrocyten in der Thrombinkomponente eines Fibrinklebers suspendiert werden,
bb) diese Suspension mit der Fibrinogenkomponente des Fibrinklebers gemischt wird,
cc) die Mischung in eine anatomisch gewünschte Form gebracht wird.

13. verfahren nach einem der Ansprüche 11- 12 **dadurch gekennzeichnet, daß** die Knochen- (a) und die Knorpelkomponente (b) vor der Verbindung getrennt in vitro kultiviert werden.

14. Verfahren nach Anspruch 13 **dadurch gekennzeichnet, daß** die Verbindung (c) von Knochen- und Knorpelkomponente mittels Fibrinklebung erfolgt.

15. Verfahren nach Anspruch 12, **dadurch gekennzeichnet, daß** während der Verfestigung des Fibrinklebers bei der Herstellung der Knorpelkomponente das noch nicht durch Osteoblasten besiedelte Trägermaterial der Knochenkomponente in die Knorpelschicht gedrückt wird, so daß es fest eingebunden wird und,
daß später die Besiedelung der Knochenkomponente durch Osteöblasten erfolgt.

16. Verfahren nach einem der Ansprüche 10-15, **dadurch gekennzeichnet, daß** es weiterhin die Herstellung einer Bandkomponente aus faserartigen Materialien und Fibroblasten umfaßt.

17. Verfahren nach einem der Ansprüche 10-16, **dadurch gekennzeichnet, daß** es weiterhin die Herstellung einer Kapselkomponente aus faserartigen, membranösen Materialien und Fibroblasten umfaßt.

18. Verfahren nach einem der Ansprüche 10-17, **dadurch gekennzeichnet, daß** an dem Trägermaterial der Knochenkomponente nach der Formgebung wenigstens eine Bandverbindungsstelle zur Anbringung von Gelenkbändern angebracht wird.

19. Verfahren nach einem der Ansprüche 10-18, **dadurch gekennzeichnet, daß** an dem Trägermaterial der Knochenkomponente wenigstens ein Kapselverbindungsareal zur Anbringung einer Gelenkkapsel angebracht wird.

20. Verfahren nach einem der Ansprüche 10-19, **dadurch gekennzeichnet, daß** wenigstens eine Bandkomponente an einer Bandverbindungsstelle der Knochenkomponente gemäß Anspruch 18 angebracht wird.

21. Verfahren nach einem der Ansprüche 10-20, **dadurch gekennzeichnet, daß** wenigstens eine Kapselkomponente an einem Kapselverbindungsareal der Knochenkomponente gemäß Anspruch 19 angebracht wird.

22. Verfahren nach einem der Ansprüche 10-21, **dadurch gekennzeichnet, daß** der Knochenkomponente Endothelzellen, ein Wachstumsfaktorprotein, oder mit einem Wachstumsfaktorgen transfizierte Zellen hinzugefügt werden.

23. Verfahren nach einem der Ansprüche 10 bis 22, **dadurch gekennzeichnet, daß** Spongiosa als Trägermaterial der Knochenkomponente verwendet wird.

24. Verfahren nach Anspruch 23, **dadurch gekennzeichnet, daß** autoklavierte, humane, allogene Spongiosa als Trägermaterial verwendet wird.

25. Verfahren nach Anspruch 10 zur Herstellung einer Knochenkomponente a), das folgende Schritte umfaßt:
aa) Transfektion von isolierten Knochenzellen oder Knochenvorläuferzellen
durch nicht-viralen Gentransfer mit wenigstens einem Gen, das für einen Wachstumsfaktor codiert;
bb) Besiedelung eines bioverträglichen Trägermaterials mit den transfizierten Zellen;
cc) Züchtung der Zell-Trägermaterial-Konstrukte in vitro.

26. Verfahren nach Anspruch 25, **dadurch gekennzeichnet, daß** die isolierten Zellen vor der Transfektion vermehrt werden.

27. Verfahren nach Anspruch 25 oder 26, **dadurch gekennzeichnet, daß** die Transfektion durch Lipofektion erfolgt.

28. Verfahren nach einem der Ansprüche 25-27 **dadurch gekennzeichnet, daß** die Trahsfektion transient ist.

29. Verfahren nach einem der Ansprüche 25-28, **dadurch gekennzeichnet, daß** das transfizierte Gen oder wenigstens eines der transfizierten Gene für einen Wachstumsfaktor codiert, der aus folgender Gruppe ausgewählt ist: EGF, bFGF, VEGF, BMP-1 bis BMP-20, TGF-β, PDGF-AA, PDGF-AB, PDGF-BB, Ang I, Ang II.

30. Verfahren nach einem der Ansprüche 25-29 **dadurch gekennzeichnet, daß** das bioverträgliche Trägermaterial auch mit nicht transfizierten Zellen besiedelt wird.

31. Verfahren nach einem der Ansprüche 25-30, **dadurch gekennzeichnet, daß** die isolierten Zellen stromale Zellen sind.

## Claims

1. A biological joint construct, produced at least partly in vitro, which comprises at least the following constituents:
a) at least one biocompatible carrier material;
b) cartilaginous tissue comprising chondrocytes and/or chondroblasts and cartilaginous substance;
c) osseous tissue comprising osteoblasts and/or osteocytes and bone substance;
cartilaginous and osseous tissue being firmly connected to one another, **characterized in that** the biological joint construct has a joint side which can have contact with another joint part and whose surface consists of cartilaginous tissue, and an anchor side which can be used for anchoring the joint construct in the bone shaft and which consists of osseous tissue.

2. The biological joint construct as claimed in claim 1, **characterized in that** the osseous tissue contains, for the improvement of angiogenesis, a growth factor protein, endothelial cells or their precursor cells, or cells transfected with a growth factor gene.

3. The biological joint construct as claimed in claim 1, **characterized in that** the anchor side has at least one cylindrical peg which can be connected to a bone shaft.

4. The biological joint construct as claimed in one of claims 1-3, **characterized in that** it additionally comprises at least one ligamentous component, which can connect two joint parts functionally to one another.

5. The biological joint construct as claimed in claim 1 or 2, **characterized in that** the joint construct is suitable for the partial replacement of a joint surface and is designed as an individual preformed construct.

6. The biological joint construct as claimed in claim 1 or 2, **characterized in that** the joint construct is suitable for the partial replacement of a joint surface and is designed as an osteochondral cylinder.

7. A biological joint replacement, **characterized in that** at least two joint constructs as claimed in one of claims 3-4 have contact with one another with their joint sides and can be anchored with their anchor sides in two different bone shafts.

8. The biological joint replacement as claimed in claim 7, **characterized in that** at least two joint constructs are connected by at least two ligamentous components.

9. The biological joint replacement as claimed in claim 7 or 8, **characterized in that** it has a joint capsule.

10. A process for the production of a biological joint construct as claimed in one of claims 1-9, which comprises the following steps:
a) production of a bone component by populating a biocompatible carrier material with osteoblasts;
b) production of a cartilaginous component by preparation of a suspension of chondrocytes in a medium or gel or by population of a biocompatible carrier substance with chondrocytes;
c) connection of the osseous and the cartilaginous component such that the carrier material is integrated into the cartilage;
d) culture of the construct in vitro, a biological crosslinkage of the combined components being achieved by stimulation of the cells to attachment and to the synthesis of their tissue-specific extracellular matrix;

11. The process as claimed in claim 10, **characterized in that** the carrier material of the bone component (a) is shaped such that it has a joint side for the acceptance of a cartilage surface and an anchor side for connection to a bone.

12. The process as claimed in claim 10 or 11, **characterized in that**, for the production of the cartilaginous component (b),
aa) chondrocytes are suspended in the thrombin component of a fibrin adhesive,
bb) this suspension is mixed with the fibrinogen component of the fibrin adhesive,
cc) the mixture is brought into an anatomically desired shape.

13. The process as claimed in one of claims 11-12, **characterized in that** the bone component (a) and the cartilaginous component (b) are cultured separately in vitro before connection.

14. The process as claimed in claim 13, **characterized in that** the connection (c) of bone component and cartilaginous component is carried out by means of fibrin adhesion.

15. The process as claimed in claim 12, **characterized in that** during the solidification of the fibrin adhesive in the production of the cartilaginous component the carrier material of the bone component, which is still not populated by osteoblasts, is pressed into the cartilaginous layer such that it is firmly bound and,
**in that** later the population of the bone component is carried out by means of osteoblasts.

16. The process as claimed in one of claims 10-15, **characterized in that** it furthermore comprises the production of a ligament component made of fibrous materials and fibroblasts.

17. The process as claimed in one of claims 10-16, **characterized in that** it furthermore comprises the production of a capsular component made of fibrous, membranous materials and fibroblasts.

18. The process as claimed in one of claims 10-17, **characterized in that** after the shaping at least one ligament connection site for the attachment of joint ligaments is attached to the carrier material of the bone component.

19. The process as claimed in one of claims 10-18, **characterized in that** at least one capsule connecting area is attached to the carrier material of the bone component for the attachment of a joint capsule.

20. The process as claimed in one of claims 10-19, **characterized in that** at least one ligament component is attached to a ligament connection site of the bone component as claimed in claim 18.

21. The process as claimed in one of claims 10-20, **characterized in that** at least one capsule component is attached to a capsule connection area of the bone component as claimed in claim 19.

22. The process as claimed in one of claims 10-21, **characterized in that** endothelial cells, a growth factor protein, or cells transfected with a growth factor gene are added to the bone component.

23. The process as claimed in one of claims 10-22, **characterized in that** spongiosa is used as a carrier material of the bone component.

24. The process as claimed in claim 23, **characterized in that** autoclaved, human, allogenic spongiosa is used as a carrier material.

25. A process as claimed in claim 10 for the preparation of a bone component a), which comprises the following steps:
aa) transfection of isolated bone cells or bone precursor cells by nonviral gene transfer using at least one gene which codes for a growth factor;
bb) population of a biocompatible carrier material with the transfected cells;
cc) culture of the cell-carrier material constructs in vitro.

26. The process as claimed in claim 25, **characterized in that** the isolated cells are proliferated before transfection.

27. The process as claimed in claim 25 or 26, **characterized in that** the transfection is carried out by lipofection.

28. The process as claimed in one of claims 25-27, **characterized in that** the transfection is transient.

29. The process as claimed in one of claims 25-28, **characterized in that** the transfected gene or at least one of the transfected genes codes for a growth factor which is selected from the following group: EGF, bFGF, VEGF, BMP-1 to BMP-20, TGF-β, PDGF-AA, PDGF-AB, PDGF-BB, Ang I, Ang II.

30. The process as claimed in one of claims 25-29, **characterized in that** the biocompatible carrier material is also populated with nontransfected cells.

31. The process as claimed in one of claims 25-30, **characterized in that** the isolated cells are stromal cells.

## Revendications

1. Structure articulaire biologique produite au moins en partie *in vitro,* comprenant au moins les constituants suivants :
a) au moins un matériau support biocompatible ;
b) du tissu cartilagineux contenant des chondrocytes et/ou des chondroblastes ainsi que de la substance cartilagineuse ;
c) du tissu osseux contenant des ostéoblastes et/ou des ostéocytes ainsi que du tissu osseux ;
dans laquelle les tissus cartilagineux et osseux sont liés solidement l'un à l'autre, **caractérisée en ce que** la structure articulaire biologique présente un côté d'articulation qui peut venir en contact avec une autre partie d'articulation et dont la surface se compose de tissu cartilagineux et un côté d'ancrage qui peut servir à ancrer la structure articulaire dans la diaphyse d'un os et qui se compose de tissu osseux.

2. Structure articulaire biologique selon la revendication 1, **caractérisée en ce que**, pour améliorer l'angiogenèse, le tissu osseux contient une protéine de facteur de croissance, des cellules endothéliales ou leurs cellules précurseurs, ou des cellules transfectées avec un gène de facteur de croissance.

3. Structure articulaire biologique selon la revendication 1, **caractérisée en ce que** le côté d'ancrage présente au moins un téton de forme cylindrique qui peut être relié à la diaphyse d'un os.

4. Structure articulaire biologique selon l'une des revendications 1 à 3, **caractérisé en ce qu'**elle comprend en plus au moins un composant ligamentaire qui peut relier de manière fonctionnelle deux parties d'articulation l'une avec l'autre.

5. Structure articulaire biologique selon la revendication 1 ou la revendication 2, **caractérisée en ce que** la structure articulaire est appropriée pour le remplacement partiel d'une surface articulaire et **en ce qu'**elle est réalisée sous la forme d'une structure unique préformée.

6. Structure articulaire biologique selon la revendication 1 ou la revendication 2, **caractérisée en ce que** la structure articulaire est appropriée pour le remplacement partiel d'une surface articulaire et **en ce qu'**elle est réalisée sous la forme d'un cylindre ostéochondral.

7. Substitut d'articulation biologique, **caractérisé en ce que** deux structures articulaires biologiques selon l'une des revendications 3-4 sont en contact l'une avec l'autre par l'intermédiaire de leurs côtés d'articulation et peuvent être ancrées par l'intermédiaire de leurs côtés d'ancrage dans les diaphyses de deux os différents.

8. Substitut d'articulation biologique selon la revendication 7, **caractérisé en ce que** deux structures articulaires au moins sont reliées par au moins deux composants ligamentaires.

9. Substitut d'articulation biologique selon la revendication 7 ou la revendication 8, **caractérisé en ce qu'**il présente une capsule articulaire.

10. Procédé de fabrication d'une structure articulaire biologique selon l'une des revendications 1 à 9, comprenant les étapes suivantes :
a) obtention d'un composant osseux par ensemencement d'un matériau support biocompatible avec des ostéoblastes ;
b) obtention d'un composant cartilagineux par préparation d'une suspension de chondrocytes dans un milieu ou un gel par ensemencement d'une substance support biocompatible avec des chondrocytes ;
c) liaison des composants osseux et cartilagineux de telle façon que le matériau support soit intégré dans le cartilage ;
d) culture de la structure *in vitro,* la stimulation des cellules en vue de la fixation et de la synthèse de leur matrice extracellulaire tissu-spécifique assurant une réticulation biologique des composants assemblés.

11. Procédé selon la revendication 10, **caractérisé en ce que** le matériau support du composant osseux (a) est formé de telle façon qu'il présente un côté d'articulation destiné à recevoir une surface cartilagineuse et un côté d'ancrage pour la liaison avec un os.

12. Procédé selon la revendication 10 ou la revendication 11, **caractérisé en ce que**, pour obtenir le composant cartilagineux (b),
aa) des chondrocytes sont mis en suspension dans le composant thrombine d'une colle fibrinique,
bb) cette suspension est mélangée avec le composant fibrinogène de la colle fibrinique,
cc) le mélange est converti en une forme souhaitée au plan anatomique.

13. Procédé selon l'une des revendications 11-12, **caractérisé en ce que** les composants osseux (a) et cartilagineux (b) sont cultivés séparément *in vitro* avant la liaison.

14. Procédé selon la revendication 13, **caractérisé en ce que** la liaison (c) des composants osseux et cartilagineux se fait par collage à la fibrine.

15. Procédé selon la revendication 12, **caractérisé en ce que**, pendant la solidification de la colle fibrinique lors de la fabrication du composant cartilagineux, le matériau support du composant osseux non encore ensemencé avec des ostéoblastes est enfoncé dans la couche de cartilage de façon à y être intégré solidement par la liaison.

16. Procédé selon l'une des revendications 10 à 15, **caractérisé en ce qu'**il comprend en outre la fabrication d'un composant ligamentaire à base de matériaux fibreux et de fibroblastes.

17. Procédé selon l'une des revendications 10 à 16, **caractérisé en ce qu'**il comprend en outre la fabrication d'un composant capsulaire à base de matériaux membraneux fibreux et de fibroblastes.

18. Procédé selon l'une des revendications 10 à 17, **caractérisé en ce que**, après le formage, au moins un site d'attache de ligament est fixé sur le matériau support du composant osseux en vue de la fixation de ligaments articulaires.

19. Procédé selon l'une des revendications 10 à 18, **caractérisé en ce qu'**au moins une zone d'attache capsulaire est fixée sur le matériau support du composant osseux en vue de la fixation d'une capsule articulaire.

20. Procédé selon l'une des revendications 10 à 19, **caractérisé en ce qu'**au moins un composant ligamentaire est fixé sur un site d'attache de ligament du composant osseux selon la revendication 18.

21. Procédé selon l'une des revendications 10 à 20, **caractérisé en ce qu'**au moins un composant capsulaire est fixé sur un site d'attache capsulaire du composant osseux selon la revendication 19.

22. Procédé selon l'une des revendications 10 à 21, **caractérisé en ce que** l'on ajoute au composant osseux des cellules endothéliales, une protéine de facteur de croissance ou des cellules transfectées avec un gène de facteur de croissance.

23. Procédé selon l'une des revendications 10 à 22, **caractérisé en ce que** l'on utilise de l'os spongieux comme matériau support pour le composant osseux.

24. Procédé selon la revendication 23, **caractérisé en ce que** l'on utilise comme matériau support de l'os spongieux allogène humain autoclavé.

25. Procédé selon la revendication 10 pour la production d'un composant osseux a), comprenant les étapes suivantes :
aa) transfection de cellules osseuses isolées ou de précurseurs de cellules osseuses par transfert génique non viral avec au moins un gène codant pour un facteur de croissance ;
bb) ensemencement du matériau support biocompatible avec les cellules transfectées ;
cc) culture de la structure cellule/matériau support *in vitro.*

26. Procédé selon la revendication 25, **caractérisé en ce que** les cellules isolées sont multipliées avant la transfection.

27. Procédé selon la revendication 25 ou la revendication 26, **caractérisé en ce que** la transfection se fait par lipofection.

28. Procédé selon l'une des revendications 25 à 27, **caractérisé en ce que** la transfection est transitoire.

29. Procédé selon l'une des revendications 25 à 28, **caractérisé en ce que** le gène transfecté ou au moins l'un des gènes transfectés code pour un facteur de croissance choisi dans le groupe constitué par les EGF, bFGF, VEGF, BMP-1 à BMP-20, TGF-β, PDGF-AA, PDGF-AB, PDGF-BB, Ang I, Ang II.

30. Procédé selon l'une des revendications 25 à 29, **caractérisé en ce que** le matériau support biocompatible est également ensemencé avec des cellules non transfectées.

31. Procédé selon l'une des revendications 25 à 30, **caractérisé en ce que** les cellules isolées sont des cellules stromales.
